(19) Europäisches Patentamt
European Patent Office
Office européen des brevets

(11) **EP 4 393 953 A1**

(12) **EUROPEAN PATENT APPLICATION**
published in accordance with Art. 153(4) EPC

(43) Date of publication:
**03.07.2024  Bulletin 2024/27**

(21) Application number: **22861769.2**

(22) Date of filing: **29.08.2022**

(51) International Patent Classification (IPC):
**C07K 16/28** (2006.01)   **C07K 16/22** (2006.01)
**A61P 35/00** (2006.01)   **A61K 39/00** (2006.01)

(52) Cooperative Patent Classification (CPC):
**A61K 39/00; A61P 35/00; C07K 16/22; C07K 16/28**

(86) International application number:
**PCT/KR2022/012859**

(87) International publication number:
**WO 2023/027561 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **27.08.2021   KR 20210114096**

(71) Applicant: **Theranotics Co., Ltd.
Hanam-si, Gyeonggi-do 12902 (KR)**

(72) Inventors:
• **JUNG, Byung Hun
Seoul 05339 (KR)**

• **LEE, Jung Wook
Seoul 06672 (KR)**
• **PARK, Dong Woon
Busan 46507 (KR)**
• **LEE, Jung Eun
Namyangju-si, Gyeonggi-do 12127 (KR)**

(74) Representative: **Klöckner, Christoph
df-mp Dörries Frank-Molnia & Pohlman
Patentanwälte Rechtsanwälte PartG mbB
Theatinerstraße 16
80333 München (DE)**

Remarks:
The complete document including Reference
Table(s) and the Sequence Listing(s) can be
downloaded from the EPO website

(54) **BISPECIFIC MOLECULE SPECIFICALLY BINDING TO B7-H3 AND TGF? AND USES THEREOF**

(57)    The present invention relates to a bispecific molecule including: B7-H3 antibody or antigen-binding fragment thereof; and a TGFβ binding portion bound thereto, more particularly, to a bispecific molecule which is bispecifically bound to B7-H3 and TGFβ and thus can be utilized as an immune checkpoint inhibitor for various diseases including cancer.

[FIG. 1]

EP 4 393 953 A1

**Description**

[Technical Field]

The present invention relates to a bispecific molecule specifically bound to B7-H3 and TGFβ.

[Background Art]

B7 homology 3 protein (B7-H3) (also referred to as CD276 and B7RP-2, which are collectively referred to as B7-H3 herein) is a type I transmembrane glycoprotein that belongs to the immunoglobulin superfamily.

Human B7-H3 includes a putative signal peptide, V-like and C-like Ig domains, a transmembrane region and a cytoplasmic domain. Exon duplication in human leads to expression of two B7-H3 isoforms having any one of an IgV-IgC-IgV-IgC-like domain including several conserved cysteine residues (4IgB7-H3 isoform) or a single IgV-IgC-like domain (2IgB7-H3 isoform). Predominant B7-H3 isoform in human tissues and cell lines is a 4IgB7-H3 isoform.

It has been reported that B7-H3 has both co-stimulatory and co-inhibitory signaling functions.

B7-H3 is not constitutively expressed on many immune cells (e.g., natural killer (NK) cells, T-cells, and antigen-presenting cells (APCs)), but its expression can be induced on these cells.

In addition, expression of B7-H3 is not restricted to the immune cells. B7-H3 transcripts are expressed in a wide spectrum of human tissues including colon, heart, liver, placenta, prostate, small intestine, testis and uterus; and in osteoblasts, fibroblasts, epithelial cells, and other cells of non-lymphoid lineage, which potentially exhibit immunological and non-immunological functions. However, protein expression in normal tissues is typically maintained at a low level, thus post-transcriptional regulation may be applied thereto.

[Summary of Invention]

[Problems to be Solved by Invention]

An object of the present invention is to provide a novel bispecific molecule, which is bispecifically bound to B7-H3 and TGFβ.

Another object of the present invention is to provide a medical use (pharmaceutical composition, therapeutic method, etc.) of a bispecific molecule specifically bound to B7-H3 and TGFβ.

[Means for Solving Problems]

1. A bispecific molecule, including: a B7-H3 antibody or antigen-binding fragment thereof; and a TGFβ binding portion bound thereto.

2. The bispecific molecule according to the above 1, wherein the B7-H3 antibody or antigen-binding fragment thereof includes a heavy chain variable region including HCDRs below and a light chain variable region including LCDRs below:

   (a) HCDRs of SEQ ID NOs: 1, 10 and 19 and LCDRs of SEQ ID NOs: 28, 37 and 45;
   (b) HCDRs of SEQ ID NOs: 2, 11 and 20 and LCDRs of SEQ ID NOs: 29, 38 and 46;
   (c) HCDRs of SEQ ID NOs: 3, 12 and 21 and LCDRs of SEQ ID NOs: 30, 39 and 47;
   (d) HCDRs of SEQ ID NOs: 4, 13 and 22 and LCDRs of SEQ ID NOs: 31, 40 and 48;
   (e) HCDRs of SEQ ID NOs: 5, 14 and 23 and LCDRs of SEQ ID NOs: 32, 41 and 49;
   (f) HCDRs of SEQ ID NOs: 6, 15 and 24 and LCDRs of SEQ ID NOs: 33, 42 and 50;
   (g) HCDRs of SEQ ID NOs: 7, 16 and 25 and LCDRs of SEQ ID NOs: 34, 43 and 51;
   (h) HCDRs of SEQ ID NOs: 8, 17 and 26 and LCDRs of SEQ ID NOs: 35, 44 and 52; or
   (i) HCDRs of SEQ ID NOs: 9, 18 and 27 and LCDRs of SEQ ID NOs: 36, 42 and 53.

3. The bispecific molecule according to the above 1, wherein the TGFβ binding portion is selected from the group consisting of an antibody or antigen-binding fragment thereof, aptamer and TGFβ receptor specifically bound to TGFβ.

4. The bispecific molecule according to the above 1, wherein the TGFβ binding portion is connected by a linker.

5. The bispecific molecule according to the above 1, wherein the TGFβ binding portion comprises an amino acid sequence of SEQ ID NO: 280.

6. The bispecific molecule according to the above 1, wherein the TGFβ binding portion is specifically bound to any one TGFβ selected from the group consisting of TGFβ1, TGFβ2 and TGFβ3.

7. The bispecific molecule according to the above 2, wherein the heavy chain variable region includes any one framework sequence selected from the group consisting of HFRs below, and the light chain variable region includes any one framework sequence selected from the group consisting of LFRs below:

(hf1) HFRs of SEQ ID NOs: 54, 63, 68 and 334;
(hf2) HFRs of SEQ ID NOs: 55, 63, 69 and 334;
(hf3) HFRs of SEQ ID NOs: 56, 64, 70 and 334;
(hf4) HFRs of SEQ ID NOs: 56, 64, 71 and 334;
(hf5) HFRs of SEQ ID NOs: 57, 64, 70 and 334;
(hf6) HFRs of SEQ ID NOs: 58, 64, 72 and 334;
(hf7) HFRs of SEQ ID NOs: 59, 65, 73 and 334;
(hf8) HFRs of SEQ ID NOs: 60, 65, 73 and 334;
(hf9) HFRs of SEQ ID NOs: 61, 66, 74 and 334;
(hf10) HFRs of SEQ ID NOs: 62, 67, 75 and 334;
(lf1) LFRs of SEQ ID NOs: 76, 82, 86 and 335;
(lf2) LFRs of SEQ ID NOs: 77, 82, 87 and 335;
(lf3) LFRs of SEQ ID NOs: 78, 83, 88 and 335;
(lf4) LFRs of SEQ ID NOs: 79, 84, 89 and 335;
(lf5) LFRs of SEQ ID NOs: 80, 84, 90 and 335;
(lf6) LFRs of SEQ ID NOs: 80, 84, 91 and 335;
(lf7) LFRs of SEQ ID NOs: 81, 85, 92 and 335;
(lf8) LFRs of SEQ ID NOs: 93, 98, 101 and 336;
(lf9) LFRs of SEQ ID NOs: 93, 98, 102 and 336;
(lf10) LFRs of SEQ ID NOs: 93, 98, 103 and 336;
(lf11) LFRs of SEQ ID NOs: 93, 98, 104 and 336;
(lf12) LFRs of SEQ ID NOs: 94, 98, 105 and 336;
(lf13) LFRs of SEQ ID NOs: 95, 99, 106 and 336;
(lf14) LFRs of SEQ ID NOs: 96, 99, 107 and 336; and
(lf15) LFRs of SEQ ID NOs: 97, 100, 108 and 336.

8. The bispecific molecule according to the above 2, wherein the heavy chain variable region is any one selected from the group consisting of SEQ ID NOs: 127, 128, 129, 130, 131, 132, 135, 142 and 152, and the light chain variable region is any one selected from the group consisting of SEQ ID NOs: 211, 221, 223, 224, 225, 231, 307, 309 and 317.

9. A gene encoding the bispecific molecule according to any one of the above 1 to 8.

10. A cell including a vector introduced therein, in which the gene of the above 9 is inserted.

11. A pharmaceutical composition for treating or preventing cancer including the bispecific molecule according to any one of the above 1 to 8.

12. The pharmaceutical composition according to the above 11, wherein the cancer is any one selected from the group consisting of lung cancer, breast cancer, ovarian cancer, uterine cancer, cervical cancer, glioma, neuroblastoma, prostate cancer, pancreatic cancer, colorectal cancer, colon cancer, head and neck cancer, leukemia, lymphoma, renal cancer, bladder cancer, gastric cancer, liver cancer, skin cancer, brain tumor, cerebrospinal cancer, adrenal tumor, melanoma, sarcoma, multiple myeloma, pancreatic neuroendocrine neoplasm, peripheral nerve sheath tumor and small cell tumor.

13. The pharmaceutical composition according to the above 11, further including an immune checkpoint inhibitor selected from the group consisting of PD-1 inhibitor, PD-L1 inhibitor, CTLA4 inhibitor, LAG3 inhibitor, TIM3 inhibitor and TIGIT inhibitor.

14. The pharmaceutical composition according to the above 1l, further including a cellular therapeutic agent selected from the group consisting of CAR-T, TCR-T, cytotoxic T lymphocytes, tumor infiltrating lymphocyte, NK and CAR-NK.

15. The bispecific molecule according to any one of the above 1 to 8, wherein the bispecific molecule is used as a medicine.

[Advantageous Effects]

The bispecific molecule of the present invention may be specifically bound to B7-H3 and TGFβ.

The bispecific molecule of the present invention may internalize TGFβ inside cells.

The bispecific molecule of the present invention may be utilized as an immune checkpoint inhibitor.

The bispecific molecule of the present invention may be administered in combination with other immune checkpoint inhibitor.

The bispecific molecule of the present invention may be administered in combination with any cellular therapeutic agent such as CAR-T, CAR-NT, etc.

The bispecific molecule of the present invention may be administered to a subject to treat a disease.

[Brief Description of Drawings]

FIG. 1 shows binding affinity according to the concentrations of #1 to #9 bispecific molecules to B7-H3.

FIG. 2 shows binding affinity according to the concentrations of #1 to #9 bispecific molecules to RKO cell line.

FIG. 3 shows binding affinity according to the concentrations of #1 to #9 bispecific molecules to RKO/B7H3 cell line.

FIG. 4 shows binding affinity according to the concentrations of #1 to #9 bispecific molecules to TGF$\beta$1.

FIG. 5 shows the binding affinity according to the concentrations of #1 to #9 bispecific molecules to TGF$\beta$2.

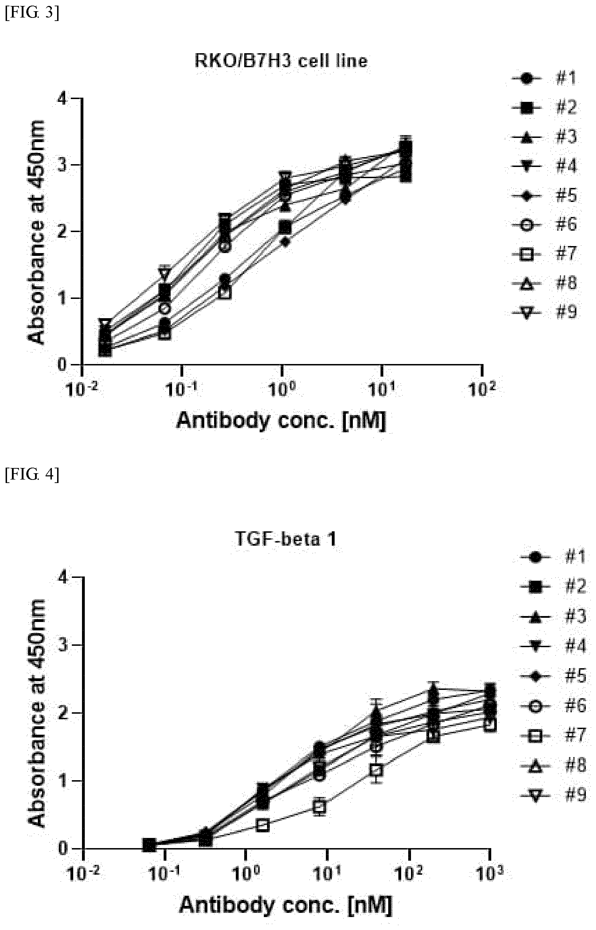

FIG. 4 shows binding affinity according to the concentrations of #1 to #9 bispecific molecules to TGF$\beta$3.

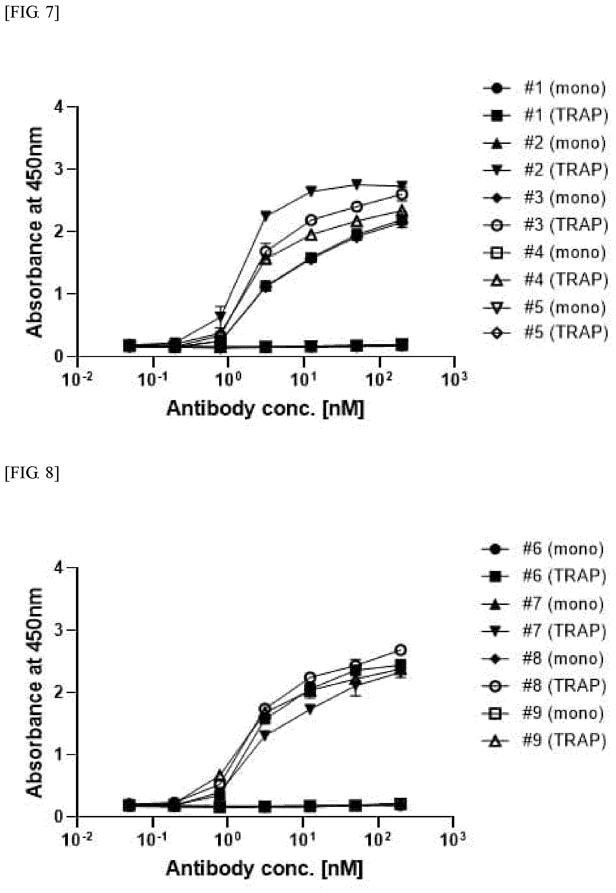

FIGS. 7 to 8 show the bispecific binding affinity according to the concentrations of #1 to #9 bispecific molecules (#1 (TRAP) to #9 (TRAP)) and B7-H3 mono antibodies (#1 (mono) to #9 (mono)) free of TGF$\beta$ binding portion to B7-H3 and TGF$\beta$. When treated with the B7-H3 mono antibodies (#1 (mono) to #9 (mono) free of TGF$\beta$ binding portion, the bispecific binding to B7-H3 and TGF$\beta$ was not confirmed.

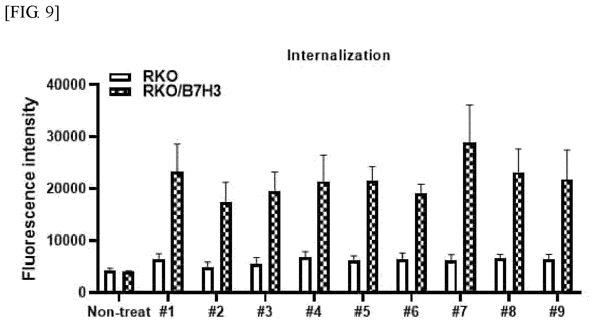

FIG. 9 illustrates measurement of a degree of internalization of bispecific molecule after treating the RKO cell line or RKO/B7H3 cell line with pHAb-labeled #1 to #9 bispecific molecules.

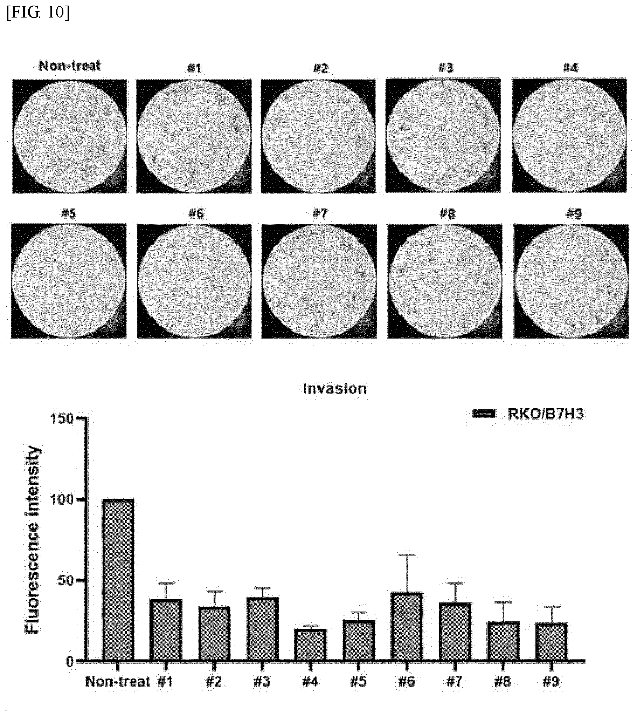

FIG. 10 illustrates invasion assay results of RKO/B7H3 cell line without any treatment (Non-treat); and RKO/B7H3 cell line treated with #1 to #9 bispecific molecules. Wherein, the degree of invasion was imaged by a microscope, while the percentage of invaded cells was calculated using Image J.

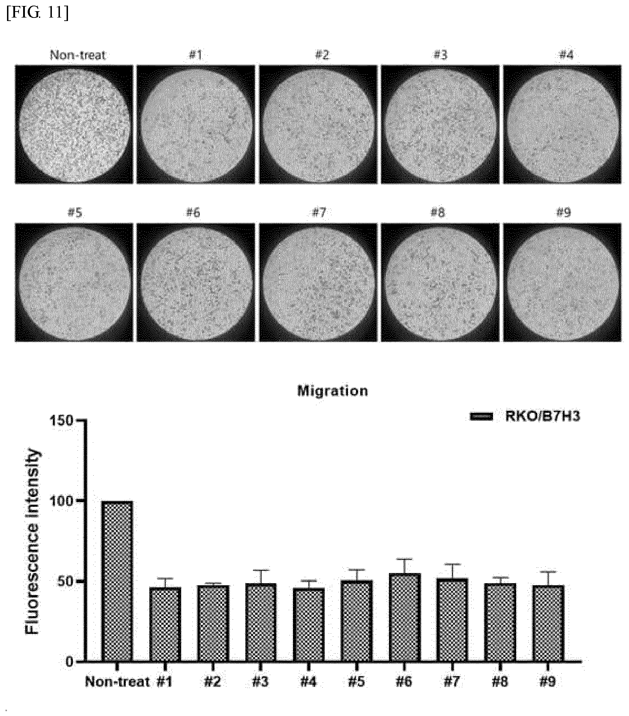

FIG. 11 illustrates migration assay results of RKO/B7H3 cell line without any treatment (Non-treat); and RKO/B7H3 cell line treated with #1 to #9 bispecific molecules. Wherein, the degree of migration was imaged by a microscope, while the percentage of OD values measured by extracting colors of cells stained with crystal violet was calculated.

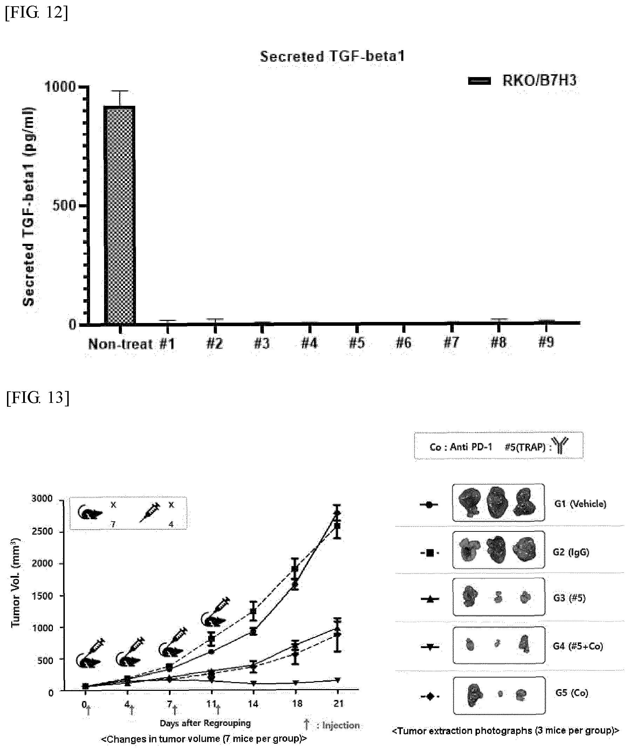

FIG. 12 illustrates TGF$\beta$ secretion assay results after treating the RKO/B7H3 cell line with #1 to #9 bispecific molecules.

FIG. 13 shows changes in tumor volume after administration of #1 to #9 bispecific molecules in mice transplanted with colon cancer cell line (CT26-TN) having over-expression of B7-H3. Wherein, G1 (vehicle) and G2 (IgG) represent the negative controls, G3 (#5) represents a #5 bispecific molecule administration group, G4 (#5+Co) represents a #5 bispecific molecule and PD-1 inhibitor (antiPD-1) combined administration group, and G5 (Co) represents a PD-1 inhibitor (antiPD-1) administration group.

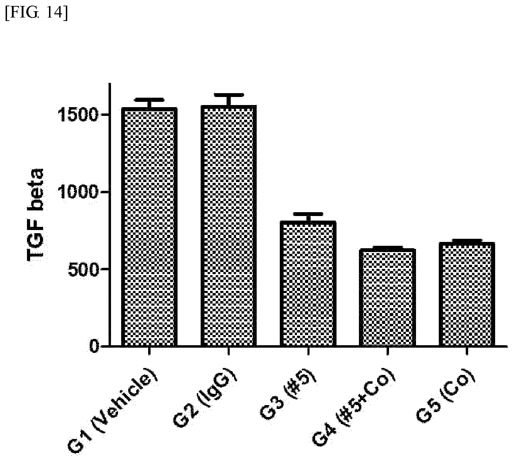

FIG. 14 shows changes in TGF$\beta$ concentration in mouse serum by #5 bispecific molecule. Wherein, G1 (vehicle) and G2 (IgG) represent the negative controls, G3 (#5) represents a #5 bispecific molecule administration group, G4 (#5+Co) represents a #5 bispecific molecule and PD-1 inhibitor (antiPD-1) combined administration group, and G5 (Co) represents a PD-1 inhibitor (antiPD-1) administration group. *: p value < 0.5 (compared to vehicle group)

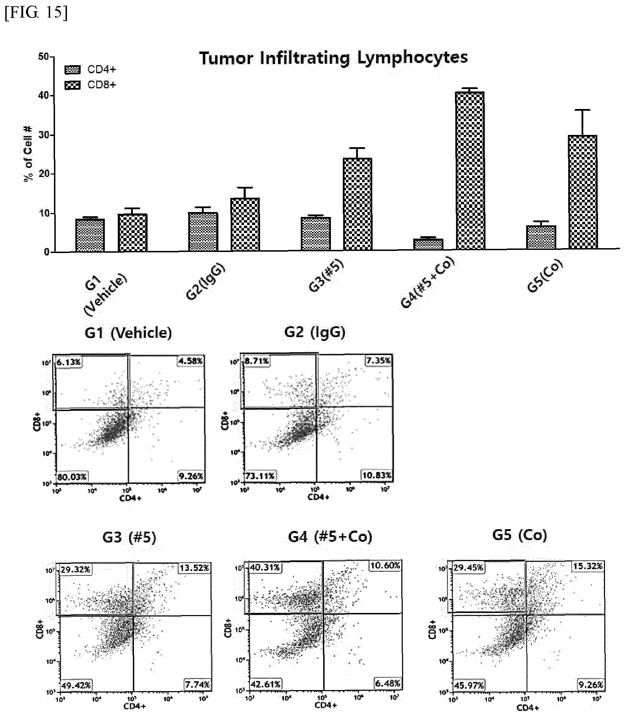

FIG. 15 shows the number of immune cells in tumor after treatment with B7-H3/TGF$\beta$ bispecific molecules. Wherein, G1 (vehicle) and G2 (IgG) represent the negative controls, G3 (#5) represents a #5 bispecific molecule administration group, G4 (#5+Co) represents a #5 bispecific molecule and PD-1 inhibitor (antiPD-1) combined administration group, and G5 (Co) represents a PD-1 inhibitor (antiPD-1) administration group.

[Mode for Carrying out Invention]

The present invention relates to a bispecific molecule, which is specifically bound to B7-H3 and TGF$\beta$.

The present invention provides a bispecific molecule, which includes: a B7-H3 antibody or antigen-binding fragment thereof; and a TGF$\beta$ binding portion bound thereto.

In the present invention, the antigen-binding fragment of the B7-H3 antibody refers to one or more fragments of the antibody that maintain the ability to specifically bind to the B7-H3.

The antibody may be of any type (e.g., IgG, IgE, IgM, IgD, IgA and IgY), class (e.g., IgG1, IgG2, IgG3, IgG4, IgA and IgA2, etc.) or subclass.

The antigen-binding fragment includes: (i) a Fab fragment which is a monovalent fragment consisting of VH, VL, CH1 and CL domains; (ii) a F(ab')$_2$ fragment which is a bivalent fragment including two Fab fragments linked by a disulfide bond in a hinge region; (iii) a Fd fragment consisting of VH and CH1 domains; (iv) an Fv fragment consisting of VL and VH domains of a single arm of an antibody; (v) a single domain or dAb fragment consisting of VH domain; (vi) an isolated complementarity determining region (CDR); and (vii) a combination of two or more isolated CDRs optionally linked by a synthetic linker.

In addition, the VL domain and the VH domain of the Fv fragment are encoded by separated genes, but they may be linked by the synthetic linker using a recombinant method so as to produce a single protein chain having a monovalent molecule (called a single chain Fv (scFv) or single chain antibody) by pairing with the VL and VH domains. This single chain antibody (scFv) is also included in the antigen-binding fragment.

The antigen-binding fragment is obtained using the conventional techniques known in the art, and functional screening of the fragment is used in the same way as for the intact antibody. Antigen binding sites may be produced by recombinant DNA technique or by enzymatic or chemical disruption of the intact immunoglobulin. The antibodies may be present as different phenotypic antibodies, for example, IgG (e.g., IgGl, IgG2, IgG3 or IgG4 subtypes), IgA1, IgA2, IgD, IgE or IgM antibodies.

The B7-H3 antibody or its antigen-binding fragment of the present invention includes a heavy chain variable region (VH) and a light chain variable region (VL).

The heavy chain variable region of B7-H3 antibody or its antigen-binding fragment of the present invention includes heavy chain complementarity determining regions (HCDRs) below, and the light chain variable region includes light chain complementarity determining regions (LCDRs) below: (a) HCDRs of SEQ ID NOs: 1, 10 and 19 and LCDRs of SEQ ID NOs: 28, 37 and 45; (b) HCDRs of SEQ ID NOs: 2, 11 and 20 and LCDRs of SEQ ID NOs: 29, 38 and 46; (c) HCDRs of SEQ ID NOs: 3, 12 and 21 and LCDRs of SEQ ID NOs: 30, 39 and 47; (d) HCDRs of SEQ ID NOs: 4, 13 and 22 and LCDRs of SEQ ID NOs: 31, 40 and 48; (e) HCDRs of SEQ ID NOs: 5, 14 and 23 and LCDRs of SEQ ID NOs: 32, 41 and 49; (f) HCDRs of SEQ ID NOs: 6, 15 and 24 and LCDRs of SEQ ID NOs: 33, 42 and 50; (g) HCDRs of SEQ ID NOs: 7, 16 and 25 and LCDRs of SEQ ID NOs: 34, 43 and 51; (h) HCDRs of SEQ ID NOs: 8, 17 and 26 and LCDRs of SEQ ID NOs: 35, 44 and 52; or (i) HCDRs of SEQ ID NOs: 9, 18 and 27 and LCDRs of SEQ ID NOs: 36, 42 and 53.

The heavy chain complementarity determining region (HCDR) consists of HCDR1, HCDR2 and HCDR3, and the light chain complementarity determining region (LCDR) consists of LCDR1, LCDR2 and LCDR3. For example, in the above sequence (a), the amino acid sequence of SEQ ID NO: 1 is HCDR1, the amino acid sequence of SEQ ID NO: 10 is HCDR2, the amino acid sequence of SEQ ID NO: 19 is HCDR3, the amino acid sequence of SEQ ID NO: 28 is LCDR1, the amino acid sequence of SEQ ID NO: 37 is LCDR2, and the amino acid sequence of SEQ ID NO: 45 is LCDR3.

The B7-H3 antibody or its antigen-binding fragment of the present invention specifically binds to the B7-H3 antigen regardless of the framework sequence, as long as it includes the above-described complementarity determining region.

The heavy chain variable region and the light chain variable region of the present invention may include various framework sequences.

The heavy chain variable region of the present invention may include, for example, any one sequence selected from the group consisting of heavy chain framework sequences (HFRs) below: (hf1) HFRs of SEQ ID NOs: 54, 63, 68 and 334; (hf2) HFRs of SEQ ID NOs: 55, 63, 69 and 334; (hf3) HFRs of SEQ ID NOs: 56, 64, 70 and 334; (hf4) HFRs of SEQ ID NOs: 56, 64, 71 and 334; (hf5) HFRs of SEQ ID NOs: 57, 64, 70 and 334; (hf6) HFRs of SEQ ID NOs: 58, 64, 72 and 334; (hf7) HFRs of SEQ ID NOs: 59, 65, 73 and 334; (hf8) HFRs of SEQ ID NOs: 60, 65, 73 and 334; (hf9) HFRs of SEQ ID NOs: 61, 66, 74 and 334; and (hf10) HFRs of SEQ ID NOs: 62, 67, 75 and 334.

The light chain variable region of the present invention may include, for example, any one sequence selected from the group consisting of light chain framework sequences (LFRs) below: (lf1) LFRs of SEQ ID NOs: 76, 82, 86 and 335; (lf2) LFRs of SEQ ID NOs: 77, 82, 87 and 335; (lf3) LFRs of SEQ ID NOs: 78, 83, 88 and 335; (lf4) LFRs of SEQ ID NOs: 79, 84, 89 and 335; (lf5) LFRs of SEQ ID NOs: 80, 84, 90 and 335; (lf6) LFRs of SEQ ID NOs: 80, 84, 91 and 335; (lf7) LFRs of SEQ ID NOs: 81, 85, 92 and 335; (lf8) LFRs of SEQ ID NOs: 93, 98, 101 and 336; (lf9) LFRs of SEQ ID NOs: 93, 98, 102 and 336; (1f10) LFRs of SEQ ID NOs: 93, 98, 103 and 336; (lf11) LFRs of SEQ ID NOs: 93, 98, 104 and 336; (lf12) LFRs of SEQ ID NOs: 94, 98, 105 and 336; (lf13) LFRs of SEQ ID NOs: 95, 99, 106 and 336; (lf14) LFRs of SEQ ID NOs: 96, 99, 107 and 336; and (lf15) LFRs of SEQ ID NOs: 97, 100, 108 and 336.

The heavy chain framework sequence (HFR) of the present invention consists of HFR1, HFR2, HFR3 and HFR4 and the light chain framework sequence (LFR) consist of LFR1, LFR2, LFR3 and LFR4. For example, in the above sequence (hf1), the amino acid sequence of SEQ ID NO: 54 is HFR1, the amino acid sequence of SEQ ID NO: 63 is HFR2, the amino acid sequence of SEQ ID NO: 68 is HFR3, and the amino acid sequence of SEQ ID NO: 334 is HFR4. In addition, for example, in the above sequence (lf1), the amino acid sequence of SEQ ID NO: 76 is LFR1, the amino acid sequence of SEQ ID NO: 82 is LFR2, the amino acid sequence of SEQ ID NO: 86 is LFR3, and the amino acid sequence of SEQ ID NO: 335 is LFR4.

The framework sequences (hf1 to hf10) of the heavy chain variable region and the framework sequences (lf1 to lf15) of the light chain variable region of the present invention may be arbitrarily combined.

The heavy and light chain complementarity determining region sequences and the heavy and light chain framework sequences of B7-H3 antibody or its antigen-binding fragment of the present invention may be arbitrarily combined. For example, any one of the heavy and light chain complementarity determining region sequences of (a) to (i), any one of the heavy chain framework sequences of (hf1) to (hf10), and any one of the light chain framework sequences (lf1) to (lf15) may be arbitrarily combined.

The heavy chain variable region of the present invention may consist of, for example, any one amino acid sequence selected from the group consisting of SEQ ID NOs: 127, 128, 129, 130, 131, 132, 135, 142 and 152.

The light chain variable region of the present invention may consist of, for example, any one amino acid sequence selected from the group consisting of SEQ ID NOs: 211, 221, 223, 224, 225, 231, 307, 309 and 317.

The antibodies or antigen-binding fragments thereof having the complementarity determining regions of (a) to (i) of the present invention may have the same or different epitopes (antigenic determinants). The epitope refers to a site of the B7-H3 antigen to which an antibody or antigen-binding fragment thereof is specifically bound. The epitopes of the antibodies or antigen-binding fragments thereof having the complementarity determining regions of (a), (d), (e), (g), (h) and (i) of the present invention are identical, and the epitopes of the antibodies or antigen-binding fragments thereof having the complementarity determining regions of (b) and (c) are identical.

In one embodiment of the present invention, #1 to #9 bispecific molecules of the bispecific molecules include heavy chain variable regions and light chain variable regions below: #1: a heavy chain variable region of SEQ ID NO: 127 and a light chain variable region of SEQ ID NO: 307; #2: a heavy chain variable region of SEQ ID NO: 128 and a light chain variable region of SEQ ID NO: 317; #3: a heavy chain variable region of SEQ ID NO: 129 and a light chain variable region of SEQ ID NO: 309; #4: a heavy chain variable region of SEQ ID NO: 130 and a light chain variable region of SEQ ID NO: 211; #5: a heavy chain variable region of SEQ ID NO: 131 and a light chain variable region of SEQ ID NO: 221; #6: a heavy chain variable region of SEQ ID NO: 132 and a light chain variable region of SEQ ID NO: 231; #7: a heavy chain variable region of SEQ ID NO: 142 and a light chain variable region of SEQ ID NO: 223; #8: a heavy chain variable region of SEQ ID NO: 152 and a light chain variable region of SEQ ID NO: 224; and #9: a heavy chain variable region of SEQ ID NO: 135 and a light chain variable region of SEQ ID NO: 225.

Among #1 to #9 bispecific molecules, B7-H3 epitopes of #1, #4, #5, #7, #8 and #9 bispecific molecules are the same to one another, while B7-H3 epitopes of #2 and #3 bispecific molecules are the same to each other.

The TGFβ binding portion of the present invention may be bound to any one selected from the group consisting of TGFβ1, TGFβ2 and TGFβ3. For example, it may be specifically bound to only TGFβ1, otherwise, may be specifically bound to all of TGFβ1, TGFβ2 and TGFβ3.

The TGFβ binding portion of the present invention may not be limited in terms of types thereof so long as it can be specifically bound to TGFβ, for example, may include an antibody or an antigen-binding fragment thereof, aptamer; or TGFβ receptor.

As the TGFβ antibody or antigen-binding fragment thereof, conventional antibody or antigen-binding fragment known to be specifically bound to TGFβ may be used. For example, "scFv capable of binding to TGFβ ligand" described in Korean Patent Laid-Open Publication No. 10-2022-0052919 may be used.

As the aptamer specifically bound to TGFβ, conventional aptamer known to be specifically bound to TGFβ may be used. For example, nucleic acid aptamer or peptide aptamer may be used.

As the TGFβ receptor, conventionally known TGFβ receptor, its variant or its fragment may be used. For example, "extracellular portion of TGF-β receptor" described in Korean Patent Laid-Open Publication No. 10-2022-0052919 may be used, or a polypeptide including an amino acid sequence SEQ ID NO: 337 may be used.

The TGFβ binding portion in the bispecific molecule of the present invention may be directly connected to B7-H3 antibody or antigen-binding fragment thereof or may be connected thereto through a linker.

The linker used herein may have any length or sequence without limitation thereof so long as it does not interrupt the binding of the bispecific molecule to B7-H3 and TGFβ. For example, the linker may have one unit sequence (e.g., GGGGS) including amino acid G and amino acid S, or have two, three, four, five or more unit sequences present continuously. Alternatively, the linker may be a polypeptide having an amino acid SEQ ID NO: 338 wherein three of unit sequences GGGGS are present continuously.

The TGFβ binding portion of the present invention may be conjugated at N-terminal or C-terminal of B7-H3 antibody or antigen-binding fragment thereof. For example, the TGFβ binding portion may be conjugated at a heavy chain C-terminal or a light chain C-terminal of B7-H3 antibody. Further, the TGFβ binding portion may be conjugated at N-terminal or C-terminal of scFv.

The bispecific molecule of the present invention may be bound to B7-H3 or TGFβ, or may be double-bound to both of B7-H3 and TGFβ.

The bispecific molecule of the present invention may have excellent binding ability to B7-H3.

The bispecific molecule of the present invention may be bound to TGFβ in a tumor-microenvironment and also bound to B7-H3 present on the surface of cell, so as to internalize TGFβ inside the cell and remove the same.

The bispecific molecule of the present invention may inhibit the generation of B7-H3.

The bispecific molecule of the present invention may contribute to T cell activation.

The present invention may provide a gene for encoding a bispecific molecule, which includes: the above-described B7-H3 antibody or antigen-binding fragment thereof; and a TGFβ binding portion bound thereto.

The gene encoding the bispecific molecule of the present invention may be included in an expression vector. The expression vector includes a promoter, a B7-H3 antibody or its antigen-binding fragment gene operably linked to the promoter, and a restriction enzyme cleavage site.

The expression vector of the present invention may be a viral vector, a naked DNA or RNA vector, a plasmid, a cosmid or phage vector, a DNA or RNA vector associated with a cationic condensing agent or a DNA or RNA vector encapsulated in the liposome.

Expression vectors of the present invention may be introduced into host cells.

The host cells of the present invention may be animal cells, plant cells, or eukaryotic cells such as eukaryotic microorganisms, and may be, for example, NS0 cells, Vero cells, Hela cells, COS cells, CHO cells, HEK293 cells, BHK cells, MDCKII cells, Sf9 cells and the like.

The host cells of the present invention may be prokaryotic cells, and may be, for example, E. coli or Bacillus subtilis.

The present invention may provide a method for preparation of a bispecific molecule, which includes: B7-H3 antibody or antigen-binding fragment thereof; and a TGFβ binding portion bound thereto, by culturing the above-described host cells. Culturing may be performed according to methods widely known in the art, and conditions such as a culture temperature, culture time, medium type and pH may be appropriately adjusted depending on the types of the cells.

The method for preparing a bispecific molecule of the present invention may further include separating, purifying, and recovering the produced bispecific molecules. For example, to the recover bispecific molecules, there are available methods such as filtration, affinity chromatography, ion exchange chromatography, hydrophobic chromatography, HPLC and the like.

The present invention may provide a pharmaceutical composition for treatment or prevention of cancer, including a bispecific molecule which includes: the above-described B7-H3 antibody or antigen-binding fragment thereof; and a TGFβ binding portion bound thereto.

The bispecific molecule of the present invention binds to B7-H3 of cancer cells in which B7-H3 is expressed to neutralize (inhibit) the activity of B7-H3, and remove B7-H3 by introducing it into the cells. Thereby, activation of immune cells may be induced, and from this, cancer may be treated.

The bispecific molecule of the present invention may inhibit the expression of B7-H3 as an immune checkpoint molecule on the surface of cancer cell so as to induce activation of immune cells, thereby treating cancer.

The bispecific molecule of the present invention may remove TGFβ so as to induce activation of immune cells, thereby treating cancer.

The cancer of the present invention may be EGFR over-expressing cancer.

The cancer of the present invention may be any one selected from the group consisting of lung cancer (small cell lung cancer and non-small cell lung cancer), breast cancer, ovarian cancer, uterine cancer, cervical cancer, glioma, neuroblastoma, prostate cancer, pancreatic cancer, colorectal cancer, colon cancer, head and neck cancer, leukemia, lymphoma, renal cancer, bladder cancer, gastric cancer, liver cancer, skin cancer, brain tumor, cerebrospinal cancer, adrenal tumor, melanoma, sarcoma (osteosarcoma and soft tissue sarcoma), multiple myeloma, pancreatic neuroendocrine neoplasm, peripheral nerve sheath tumor and small cell tumor.

The pharmaceutical composition of the present invention may be more effective for solid tumor.

The pharmaceutical composition of the present invention may further include a pharmaceutically acceptable carrier, and may be formulated with the carrier. The term "pharmaceutically acceptable carrier" refers to a carrier or diluent that does not stimulate the organism and does not inhibit biological activities and properties of the administered compound.

Pharmaceutically acceptable carriers for liquid compositions include saline, sterile water, Ringer's solution, buffered saline, albumin injectable solutions, dextrose solution, maltodextrin solution, glycerol, ethanol, and a mixture of one or more of these components. If necessary, other conventional additives such as antioxidants, buffers, and bacteriostats may be added to the carrier. In addition, diluents, dispersants, surfactants, binders and lubricants may also be additionally added to formulate the pharmaceutical composition into injectable formulations, pills, capsules, granules or tablets such as aqueous solutions, suspensions, emulsions and the like.

The pharmaceutical composition of the present invention is not limited in the formulation, and may be prepared, for example, in oral or parenteral formulations. More specifically, the formulations include oral, rectal, nasal, topical (including the cheek and sublingual), subcutaneous, vaginal or intramuscular, subcutaneous and intravenous administration. Alternatively, forms suitable for administration by inhalation or insufflations may also be included.

The pharmaceutical composition of the present invention is administered to a subject in a pharmaceutically effective amount. The effective amount may be determined depending on types and severity of disease of the patient, activity of drug, sensitivity to drug, administration time, administration route and rate of release, duration of treatment, factors including concurrent drugs, and other factors well known in the medical field.

The dosage of the pharmaceutical composition of the present invention may vary depending on the weight, age, gender, health conditions or diet of a patient, administration time, administration method, excretion rate and severity of the disease. The appropriate dosage may vary depending on, for example, an amount of drug accumulated in the patient's body and/or the efficacy of the active ingredient of the present invention used.

Generally, the amount may be calculated on the basis of $EC_{50}$, which is generally determined to be effective in *in vivo* animal models and *in vitro,* for example, from 0.01 μg to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

The pharmaceutical composition of the present invention may further include an immune checkpoint inhibitor. For example, the pharmaceutical composition of the present invention may further include an immune checkpoint inhibitor selected from the group consisting of PD-1 inhibitor, PD-L1 inhibitor, CTLA4 inhibitor, LAG3 inhibitor, TIM3 inhibitor and TIGIT inhibitor.

The pharmaceutical composition of the present invention may further include a cellular therapeutic agent. For example, the pharmaceutical composition of the present invention may further include a cellular therapeutic agent selected from the group consisting of chimeric antigen receptor T (CAR-T) cells, T cell receptor T (TCR-T) cells, cytotoxic T lymphocyte (CTL), tumor infiltrating lymphocyte (TIL), natural killer (NK) cells and chimeric antigen receptor-natural killer (CAR-NK) cells.

The pharmaceutical composition of the present invention may further include a component to maintain or increase the solubility and absorption of the active ingredient. In addition, the pharmaceutical composition may further include chemotherapeutic agents, anti-inflammatory agents, antiviral agents, immunomodulators and the like.

Further, the pharmaceutical composition of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

The present invention may provide a method for treatment of cancer, including a process of administering a gene encoding a bispecific molecule, which includes: B7-H3 antibody or antigen-binding fragment thereof; and a TGFβ binding portion bound thereto, to a subject. The cancer possibly treated herein may be as described above.

The bispecific molecule of the present invention, or the gene encoding the same, may be administered to a human subject for therapeutic purposes.

The bispecific molecule of the present invention, or the gene encoding the same, may be administered to a non-human mammal expressing B7-H3 for veterinary purposes or as an animal model of human diseases.

The present invention may provide a bispecific molecule useable as a medicine, which includes: B7-H3 antibody or antigen-binding fragment thereof; and a TGFβ binding portion bound thereto.

The bispecific molecule of the present invention may be administered to a subject suffering from "a disease or disorder in which B7-H3 activity is detrimental" for therapeutic purposes.

The "disease or disorder in which B7-H3 activity is detrimental" of the present invention includes diseases and disorders in which the presence of B7-H3 in the subject suffering from a specific disease or disorder has been turn out or suspected to be a factor responsible for the pathophysiology of the disorder or contributing to the worsening of the disorder.

The medicament of the present invention may be an anticancer drug for treatment of cancer. The types of cancer are as described above.

SEQ ID NOs: 1 to 27 are complementarity determining region (HCDR) sequences among heavy chain variable regions of B7-H3 antibody or antigen-binding fragment thereof. In particular, SEQ ID NOs: 1 to 9 are HCDR1 sequences, SEQ ID NOs: 10 to 18 are HCDR2 sequences, and SEQ ID NOs: 19 to 27 are HCDR3 sequences.

SEQ ID NOs: 28 to 53 are complementarity determining region (LCDR) sequences among light chain variable regions of B7-H3 antibody or antigen-binding fragment thereof. In particular, SEQ ID NOs: 28 to 36 are LCDR1 sequences, SEQ ID NOs: 37 to 44 are LCDR2 sequences, and SEQ ID NOs: 45 to 53 are LCDR3 sequences.

SEQ ID NOs: 54 to 75 are framework sequences (HFR) among heavy chain variable regions of B7-H3 antibody or antigen-binding fragment thereof. In particular, SEQ ID NOs: 54 to 62 are HFR1 sequences, SEQ ID NOs: 63 to 67 are HFR2 sequences, and SEQ ID NOs: 68 to 75 are HFR3 sequences.

SEQ ID NOs: 76 to 92 are framework sequences (LFR) among kappa light chain variable regions of B7-H3 antibody or antigen-binding fragment thereof. In particular, SEQ ID NOs: 76 to 81 are LFR1 sequences, SEQ ID NOs: 82 to 85 are LFR2 sequences, and SEQ ID NOs: 86 to 92 are LFR3 sequences.

SEQ ID NOs: 93 to 108 are framework sequences (LFR) among lambda light chain variable regions of B7-H3 antibody or antigen-binding fragment thereof. In particular, SEQ ID NOs: 93 to 97 are LFR1 sequences, SEQ ID NOs: 98 to 100 are LFR2 sequences, and SEQ ID NOs: 101 to 108 are LFR3 sequences.

SEQ ID NOs: 109 to 198 are heavy chain variable regions (VH) of B7-H3 antibody or antigen-binding fragment thereof in which the HCDR and HFR sequences described above are included.

SEQ ID NOs: 199 to 333 are light chain variable regions (VL) of B7-H3 antibody or antigen-binding fragment thereof in which the LCDR and LFR sequences described above are included.

SEQ ID NO: 334 is HFR4 sequence among heavy chain variable region framework sequences of B7-H3 antibody or antigen-binding fragment thereof. SEQ ID NOs: 335 and 336 are LFR4 sequences among light chain variable region framework sequences of B7-H3 antibody or antigen-binding fragment thereof.

SEQ ID NO: 337 is an amino acid sequence of TGFβ receptor according to an embodiment of the present invention. SEQ ID NO: 338 is an amino acid sequence according to an embodiment of the present invention.

SEQ ID NOs: 339 to 347 are each sequence in which "B7-H3 antibody heavy chain sequence", "linker sequence (SEQ ID NO: 338)", and "TGFβ receptor sequence (SEQ ID NO: 337) of #1 to #9 bispecific molecules are connected in order. The B7-H3 antibody heavy chain sequence is a sequence in which a heavy chain constant region sequence of SEQ ID NO: 348 is connected to the end of the heavy chain variable region of the B7-H3 antibody described above.

Hereinafter, the present invention will be described in more detail through examples.

## EXAMPLE

With regard to the bispecific molecules according to an embodiment of the present invention, experimental results for B7-H3 binding force, TGFβ binding force, TGFβ cell internalization ability, cancer cell infiltration, migration inhibitory ability, or the like are summarized in the following examples. In the tables and description: #1 indicates a bispecific molecule which includes (B7-H3 antibody heavy chain)-(linker)-(TGFβ binding portion) of SEQ ID NO: 339, and B7-H3 antibody light chain variable region of SEQ ID NO: 307; #2 indicates a bispecific molecule which includes (B7-H3 antibody heavy chain)-(linker)-(TGFβ binding portion) of SEQ ID NO: 340, and B7-H3 antibody light chain variable region of SEQ ID NO: 317; #3 indicates a bispecific molecule which includes (B7-H3 antibody heavy chain)-(linker)-(TGFβ binding portion) of SEQ ID NO: 341, and B7-H3 antibody light chain variable region of SEQ ID NO: 309; #4 indicates a bispecific molecule which includes (B7-H3 antibody heavy chain)-(linker)-(TGFβ binding portion) of SEQ ID NO: 342, and B7-H3 antibody light chain variable region of SEQ ID NO: 211; #5 indicates a bispecific molecule which includes (B7-H3 antibody heavy chain)-(linker)-(TGFβ binding portion) of SEQ ID NO: 343, and B7-H3 antibody light chain variable region of SEQ ID NO: 221; #6 indicates a bispecific molecule which includes (B7-H3 antibody heavy chain)-(linker)-(TGFβ binding portion) of SEQ ID NO: 344, and B7-H3 antibody light chain variable region of SEQ ID NO: 231; #7 indicates a bispecific molecule which includes (B7-H3 antibody heavy chain)-(linker)-(TGFβ binding portion) of SEQ ID NO: 345, and B7-H3 antibody light chain variable region of SEQ ID NO: 223; #8 indicates a bispecific molecule which includes (B7-H3 antibody heavy chain)-(linker)-(TGFβ binding portion) of SEQ ID NO: 346, and B7-H3 antibody light chain variable region of SEQ ID NO: 224; and #9 indicates a bispecific molecule which includes (B7-H3 antibody heavy chain)-(linker)-(TGFβ binding portion) of SEQ ID NO: 347, and B7-H3 antibody light chain variable region of SEQ ID NO: 225.

Further, in the tables and description, the case where #1, #2, etc. are represented and the case where #1 (TRAP), #2 (TRAP), etc. are represented exhibit the bispecific molecules according to an embodiment of the present invention. On the other hand, the case where #1 (mono), #2 (mono), etc. are represented refers to a mono antibody not having TGFβ binding portion.

## Example 1: Binding force test of B7-H3 using ELISA method

This experiment was conducted to confirm the binding force of the B7-H3/TGFβ bispecific molecules to B7-H3 protein.

## Experimental method

The binding force according to the concentrations of #1 to #9 bispecific molecules to B7-H3 was confirmed by the following method.

After coating a plate with recombinant human B7-H3 protein (R&D Systems, Cat# 1027-B3-100) (30 μL, 20 nM) in 1 × PBS solution, the plate was covered and coated overnight at 2 to 8 °C. Thereafter, the wells were washed once with 150 μL PBS per well, and blocked with 120 μL of blocking buffer (1 × PBS-T w/3% BSA) per well for 2 hours at room temperature. The blocking buffer was discarded and 30 μL of antibody solution was added using a series of dilution solutions (using the blocking buffer, #1 to #9 bispecific molecules were diluted in serial by two times; 2-fold serial dilution), and reacted at room temperature for 1 hour, then the wells were washed three times with 150 μL of wash buffer per well. 30 μL of HRP conjugate of anti-HA Tag antibody diluted in the blocking buffer was added to each well, and reacted at room temperature for 1 hour. Thereafter, the wells were washed three times with 150 μL of washing buffer per well, the HRP reaction was developed using TMB, the reaction was stopped with 1 N HCl, and then the optical density (OD) was measured at 450 nm.

## Results

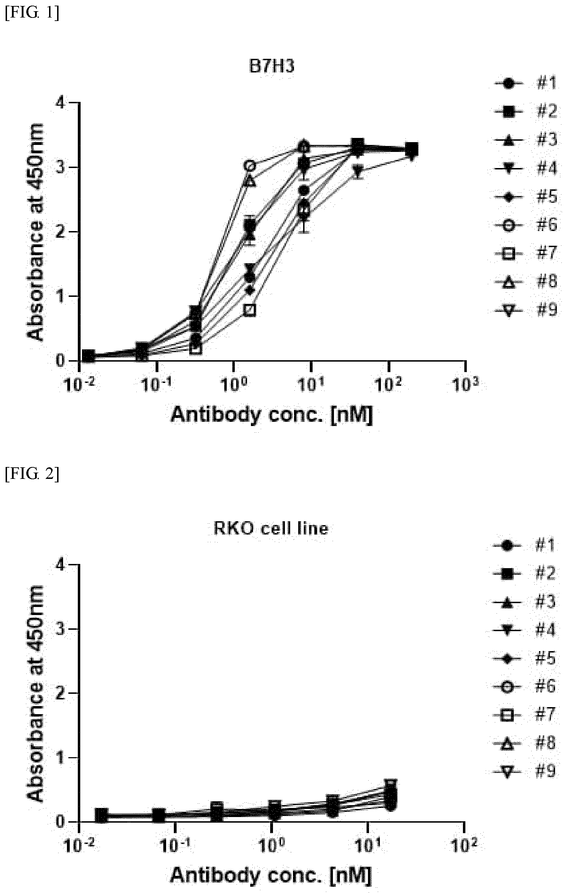

The binding force according to the concentrations of #1 to #9 bispecific molecules to B7-H3, and the concentrations of the respective bispecific molecules that allow 50% of B7-H3 to exist in an antigen-antibody bound state when #1 to #9 bispecific molecules are treated ($EC_{50}$) were checked (FIG. 1). It was confirmed that #1 to #9 bispecific molecules specifically bind to B7-H3 with excellent binding force.

[TABLE 1]

| Item | | $EC_{50}(nM)$ |
|---|---|---|
| Bispecific molecule | #1 | 27.52 |
| | #2 | 3.194 |
| | #3 | 5.944 |
| | #4 | 29.33 |
| | #5 | 10.16 |
| | #6 | 1.340 |
| | #7 | 12.23 |
| | #8 | 1.724 |
| | #9 | 6.112 |

## Example 2: Binding force test of B7-H3 antibody using cell ELISA method

This experiment was conducted to confirm the binding force of the B7-H3/TGFβ bispecific molecules to B7-H3 expressed on the cell membrane.

## Experimental method

### 2.1. Preparation of reagents

1 × PBS and 1 × PBS-T (0.05% Tween 20) were prepared. A blocking buffer was prepared so that BSA was 3% BSA in 1 × PBS-T (0.05% Tween 20). An antibody dilution buffer was prepared so that BSA was 1% BSA in 1 × PBS-T (0.05% Tween 20).

### 2.2. Cell harvesting and seeding

After harvesting cells from RKO cell line, and RKO/B7H3 cell line, the cells were diluted with a culture medium (10% FBS added) so that they could be seeded with $3 \times 10^4$ cells, 100 μL/well to adjust the cell concentration. After seeding at 100 μL/well in a cell culture plate, 96-well plate, followed by culturing overnight in an incubator at 37 °C and 5% $CO_2$.

### 2.3. Cell fixation and blocking

1) 100 μL of 8% paraformaldehyde solution was added to the 96-well plate, and centrifuged at 300 g for 10 minutes, then fixed at room temperature for a total of 20 minutes including the centrifugation time.
2) Thereafter, the fixation solution was removed, and the wells were washed by adding 1 × PBS at 250 μL/well.
3) After washing, 250 μL/well of blocking buffer was added and incubated at room temperature for 1 hour. After removing the blocking buffer, 1 × PBS-T was added at 250 μL/well to wash the wells again, and the PBS-T remaining in the wells was swept off (this washing process was repeated three times).

### 2.4. Antibody reaction

After diluting #1 to #9 bispecific molecules by serial dilution method, the diluted samples (bispecific molecules) at the concentrations shown in Table 2 below were dispensed into a 96-well plate in duplicate by 100 μL, so that the bispecific molecules were bound at room temperature for 2 hours. Thereafter, washing was three times performed.

The concentrations of the bispecific molecules used in this experiment are shown in Table 2 below.

[TABLE 2]

| Item | #1 to #9 bispecific molecules |
|---|---|
| **Concentration (RKO and RKO/B7H3 cell ELISA)** | 4 fold serial dilution from 2.5 μg/mL |

**2.5. Detection antibody reaction**

Peroxidase-AffiniPure Rabbit Anti-Human IgG, F(ab')2 fragment specific antibody was diluted at a ratio of 1:5,000 using an antibody dilution buffer, and 100 μL was dispensed into each well, and reacted at room temperature for 1 hour. Thereafter, the wells were washed three times and 100 μL of 1-step TMB substrate solution was dispensed into each well, then reacted at room temperature for 10 minutes by eliminating light. After 10 minutes, 50 μL of 1 N HCl was added to each well to stop the TMB reaction, and the OD values were measured at 450 nm.

**Results**

Binding affinity according to the concentrations of #1 to #9 bispecific molecules to RKO and RKO/B7H3 cell lines was checked.

For RKO cell line without over-expression of B7-H3, all of #1 to #9 bispecific molecules showed weak binding force (FIG. 2). However, for RKO/B7H3 cell line with over-expression of B7-H3 on a cell membrane, all of #1 to #9 bispecific molecules exhibited strong binding force (FIG. 3 and Table 3).

Table 3 below shows the $EC_{50}$ concentrations of each bispecific molecule to RKO/B7H3 cells.

[TABLE 3]

| Item | | $EC_{50}$ (nM) |
|---|---|---|
| **Bispecific molecule** | #1 | 0.443 |
| | #2 | 0.099 |
| | #3 | 0.202 |
| | #4 | 0.180 |
| | #5 | 0.940 |
| | #6 | 0.188 |
| | #7 | 0.704 |
| | #8 | 0.174 |
| | #9 | 0.104 |

**Example 3: TGFβ1, TGFβ2 and TGFβ3 binding force test using ELISA method**

In order to investigate the binding force of B7-H3/TGFβ bispecific molecule to TGFβ (TGFβ1, TGFβ2 and TGFβ3), this experiment was conducted.

**Experimental method**

**3.1. Test for binding force to TGFβ1 or TGFβ3**

After coating a plate with each of recombinant human TGFβ1 protein (R&D Systems, Cat# 7754-BH-025/CF) and TGFβ3 protein (R&D Systems, Cat# 8420-B3-025/CF) (30 μL, 0.5 μg/ml) in 1 × PBS solution, the plate was covered and cultured overnight at 2 to 8 °C. Thereafter, the wells were washed once with 150 μL PBS per well, and blocked with 120 μL of blocking buffer (1 × PBS-T w/3% BSA) per well for 2 hours at room temperature. The blocking buffer was discarded and 30 μL solution containing #1 to #9 bispecific molecules (5-fold serial dilution) was added, followed by reaction for 1 hour at room temperature. Then, the wells were washed three times with 150 μL PBS per well. 30 μL of HRP conjugated anti-Human IgG1 Fc Ab diluted in the blocking buffer was added to each well, and reacted at room temperature for 1 hour. Thereafter, the wells were washed three times with 150 μL of washing buffer per well, the HRP reaction was developed using TMB, and the reaction was stopped with 30 μL 1 N HCl. Then, the optical density (OD) was measured at 450 nm.

### 3.2. Test for binding force to TGFβ2

After coating a plate with 1 × PBS solution containing #1 to #9 bispecific molecules (30 μL, 0.5 μM), the plate was covered and cultured overnight at 2 to 8 °C. Thereafter, the wells were washed once with 150 μL PBS per well, and blocked with 120 μL of blocking buffer (1 × PBS-T w/3% BSA) per well for 2 hours at room temperature. The blocking buffer was discarded, recombinant human TGFβ2 (R&D Systems, Cat# 302-B2-010/CF) was 4-fold diluted in serial from 30 nM. After adding each diluted solution to the wells by 30 μL per well, it was reacted at room temperature for 1 hour. Then, the wells were washed three times with 150 μL of washing buffer (1 × PBS-T) per well, followed by diluting Biotinylated-TGF-beta2 antibody combined with the recombinant human TGFβ2 protein in the blocking buffer. After adding the diluted solution to the wells by 30 μL per well, it was reacted at room temperature for 1 hour. Further, after washing the wells three times with 150 μL of washing buffer (1 × PBS-T) per well, 30 μL of streptavidin-HRP diluted in the blocking buffer was added to each well, and reacted at room temperature for 1 hour. After washing the wells three times with 150 μL of washing buffer (1 × PBS-T) per well, HRP reaction was developed using TMB, and the reaction was stopped with 30 μL 1 N HCl. Then, the optical density (OD) was measured at 450 nm.

### Results

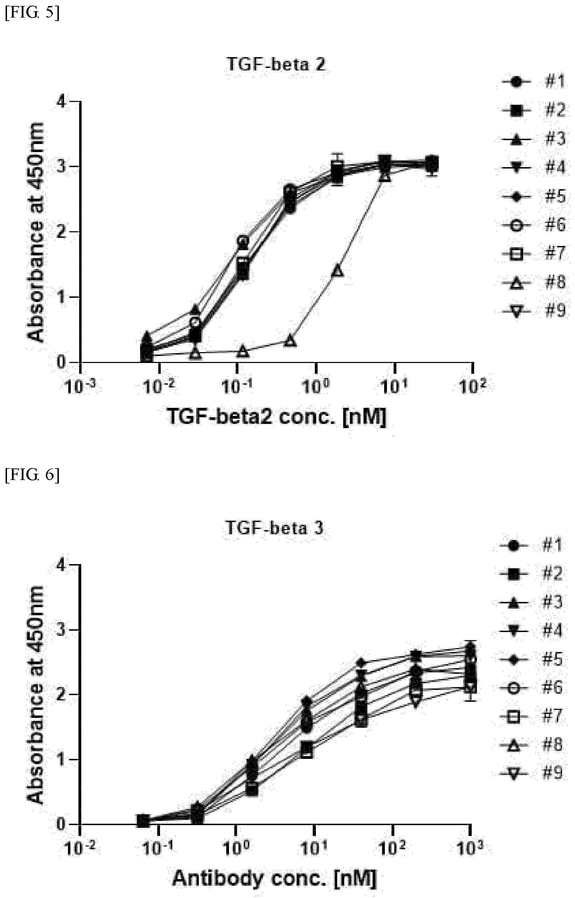

The binding force according to the concentrations of #1 to #9 bispecific molecules to TGFβ1 and TGFβ3 proteins, and the concentrations of the respective antibodies ($EC_{50}$) (TGFβ1, TGFβ3) and TGFβ 2 ($EC_{50}$) that allow 50% thereof to exist in an antigen-antibody bound state when #1 to #9 bispecific molecules are treated were checked (FIGS. 4 and 6, Tables 4 and 6).

Further, the binding force according to the concentrations of #1 to #9 bispecific molecules to TGFβ2 protein, and the concentration of TGFβ2 protein that allows 50% thereof to exist in an antigen-antibody bound state when TGFβ2 protein is treated was checked (FIG. 5, Table 5).

From these results, it was confirmed that #1 to #9 bispecific molecules specifically bind to TGFβ1, TGFβ2 and TGFβ3 with excellent binding force.

Table 4 shows the concentration of bispecific molecule ($EC_{50}$) that allows 50% thereof to exist in an antigen-antibody bound state with TGFβ1 when #1 to #9 bispecific molecules are treated.

[TABLE 4]

| Item | | $EC_{50}$(nM) |
|---|---|---|
| Bispecific molecule | #1 | 3.838 |
| | #2 | 5.833 |
| | #3 | 4.394 |
| | #4 | 2.018 |
| | #5 | 3.624 |
| | #6 | 9.077 |
| | #7 | 24.80 |
| | #8 | 3.414 |
| | #9 | 3.940 |

Table 5 shows the concentration of TGFβ2 ($EC_{50}$) that allows 50% thereof to exist in an antigen-antibody bound state with TGFβ2 when the antigen is treated with #1 to #9 bispecific molecules.

[TABLE 5]

| Item | | EC$_{50}$(nM) |
|---|---|---|
| **Bispecific molecule** | #1 | 0.136 |
| | #2 | 0.140 |
| | #3 | 0.076 |
| | #4 | 0.130 |
| | #5 | 0.143 |
| | #6 | 0.083 |
| | #7 | 0.120 |
| | #8 | 2.080 |
| | #9 | 0.143 |

Table 6 shows the concentration of the bispecific molecules (EC$_{50}$) that allows 50% thereof to exist in an antigen-antibody bound state with TGFβ3 when #1 to #9 bispecific molecules are treated.

[TABLE 6]

| Item | | EC$_{50}$(nM) |
|---|---|---|
| **Bispecific molecule** | #1 | 4.716 |
| | #2 | 7.918 |
| | #3 | 3.804 |
| | #4 | 3.323 |
| | #5 | 3.141 |
| | #6 | 4.558 |
| | #7 | 8.089 |
| | #8 | 2.939 |
| | #9 | 5.978 |

### Example 4: Test for co-binding force of B7-H3 and TGFβ1 by ELISA method

In order to investigate the binding force of B7-H3/TGFβ bispecific molecule to TGFβ3 and TGFβ1, this experiment was conducted.

### Experimental method

After coating a plate with the recombinant human TGFβ1 protein (30 μL, 0.5 μg/ml) in 1 × PBS solution, the plate was covered and cultured overnight at 2 to 8 °C. Thereafter, the wells were washed once with 150 μL PBS per well, and blocked with 120 μL of blocking buffer (1 × PBS-T w/3% BSA) per well for 2 hours at room temperature. The blocking buffer was discarded and 4-fold diluted antibody solution was added by 30 μL, followed by reaction for 2 hour at room temperature. Then, the wells were washed three times with 150 μL of washing buffer (1 × PBS-T) per well.

Thereafter, 30 μL of His-tag human B7-H3 protein diluted in the blocking buffer was added to each well, and cultured at room temperature for 2 hours. Thereafter, the wells were washed three times with 150 μL of washing buffer per well. 30 μL of HRP conjugate of anti-His tag antibody diluted in the blocking buffer was added to each well, and reacted at room temperature for 1 hour. Thereafter, the wells were washed three times with 150 μL of washing buffer per well, the HRP reaction was developed using TMB, and the reaction was stopped with 30 μL 1 N HCl. Then, the optical density (OD) was measured at 450 nm

**Results**

It was confirmed that #1 to #9 bispecific molecules (#1 (TRAP) to #9 (TRAP)) are simultaneously bound to B7-H3 protein and TGFβ1 protein. Further, it was also confirmed that B7-H3 mono antibodies (#1 (mono) to #9 (mono)) free of TGFβ binding portion are not bound to TGFβ1.

Tables 7 and 8 show the concentrations of the bispecific molecules ($EC_{50}$) that allow 50% thereof to exist in an antigen-antibody bound state with B7-H3 and TGFβ1 when #1 to #9 bispecific molecules (#1 (TRAP) to #9 (TRAP)) are treated.

[TABLE 7]

| Item | $EC_{50}$(nM) |
|---|---|
| #1(mono) | - |
| #1(TRAP) | 3.825 |
| #2(mono) | - |
| #2(TRAP) | 1.425 |
| #3(mono) | - |
| #3(TRAP) | 2.111 |
| #4(mono) | - |
| #4(TRAP) | 1.958 |
| #5(mono) | - |
| #5(TRAP) | 3.744 |

[TABLE 8]

| Item | $EC_{50}$(nM) |
|---|---|
| #6(mono) | - |
| #6(TRAP) | 2.208 |
| #7(mono) | - |
| #7(TRAP) | 3.147 |
| #8(mono) | - |
| #8(TRAP) | 1.984 |
| #9(mono) | - |
| #9(TRAP) | 1.559 |

**Example 5: Cell internalization test**

This experiment was conducted to confirm B7-H3 internalization ability of the B7-H3/TGFβ bispecific molecule.

**5.1. Antibody-pHAb amine reactive dye conjugation**

After dissolving 0.084 g of sodium bicarbonate in distilled water, the pH was adjusted to 8.5 using a pH meter, and the final volume was adjusted to 100 mL, followed by filtering impurities with a syringe filter, to prepare an equilibration buffer (10 mM sodium bicarbonate buffer, pH 8.5). Thereafter, the antibody buffer was replaced with the equilibration buffer using a desalting column. The storage solution of the column was removed, and the bottom closure of the column was removed to change the storage solution of the antibody to the equilibration buffer, and then put into a 1.5 mL microcentrifuge tube.

Centrifugation was performed at 1,500 g for 1 minute, then the microcentrifuge tube was replaced with a new one. Then, washing was performed. 300 μL of equilibration buffer was put into the tube, then centrifugation was performed at 1,500 g for 1 minute twice. After performing the process twice, 300 μL of equilibration buffer was put into the tube, followed by centrifugation at 1,500 g for 2 minutes. Then, 70 μL of each antibody was put and centrifuged at 1,500 g for 2 minutes.

Amine-reactive dye was taken out at -80°C, centrifuged at 14,000 g for 10 seconds to settle down, and DMSO and distilled water were mixed at a ratio of 1:1. Next, 25 μL of the mixture was put into 10 mg/mL, and vortexed for 3 minutes to fully dissolve.

Thereafter, antibody-pHAb amine reactive dye conjugation was performed.

1.2 μL of pHAb amine-reactive dye was put into 100 μg of antibody, then was slowly mixed for 1 hour at room temperature. Then, the antibody and pHAb amine reactive dye conjugation reagent were put into the desalting column, and unreacted dye was removed by centrifugation at 1,500 g for 2 minutes. The concentrations of the pHAb amine dye and the conjugated antibody were calculated using the following equations:

$$\text{Antibody Concentration(mg/mL)} = \frac{A_{280} - (A_{532} \times 0.256)}{1.4}$$

$$\text{Dye} - \text{to} - \text{Antibody Ratio(DAR)} = \frac{(A_{532} \times 150,000)}{\text{Ab Concentration}\left(\frac{mg}{ml}\right) \times 75,000}$$

(wherein, molecular weight of antibody = 150,000, extinction coefficient of pHAb reactive dye = 75,000, and correction factor for pHAb reactive dye = 0.256).

### 5.2. Cell seeding

After harvesting the RKO cell line and RKO/B7H3 cell line, the number of cells was counted. The cells were suspended at a cell concentration of $3 \times 10^5$ cells/mL using a culture medium.

The cells were dispensed into a 96-well black, clear-bottom plate by 100 μL so as to be $3 \times 10^4$ cells per well, and incubated for 24 hours in an incubator at 37 °C and 5% $CO_2$.

### 5.3. Conjugation of primary antibody to pHAb amine-labeled secondary antibody

4 μg/mL of primary antibody (control IgG, #1 to #9 bispecific molecules) and pHAb amine-labeled secondary antibody were put into a RPMI1640 (phenol free, serum free) medium at a ratio of 1:4 and mixed. Then, the mixture was put into a thermostatic water bath at 37 °C and reacted for 1 hour.

### 5.4. Conjugated antibody treatment

After removing the culture medium put when seeding the cells, 100 μL of a solution in which the primary antibody and the pHAb amine-labeled secondary antibody were conjugated was dispensed into each well. Then, a reaction was performed for 24 hours in an incubator at 37 °C and 5% $CO_2$.

### 5.5. Fixation and washing

The culture solution treated with the conjugated antibody was removed, and 4% paraformaldehyde was dispensed by 100 μL. The 96-well plate was centrifuged at 300 g for 10 minutes. After centrifugation, a reaction was performed at room temperature for 10 minutes. Thereafter, 250 μL of 1 × PBS was added per well and washed three times, then 100 μL of 1 × PBS was added per well.

### 5.6. Analysis

Fluorescence intensities were measured by OD values of Ex 520 nm/Em 565 nm using a microplate reader.

Results

With regard to RKO cell lines without over-expression of B7-H3, the groups treated with #1 to #9 bispecific molecules did not show significant difference in fluorescent intensity, accordingly, it could be seen that internalization occurred very little (FIG. 9)

On the other hand, with regard to RKO/B7H3 cell lines with over-expression of B7-H3, the groups treated with #1 to #9 bispecific molecules exhibited greatly increased fluorescent intensity. That is, it was confirmed that #1 to #9 bispecific molecules are bound to B7-H3 over-expressed on the surface of cell and have excellent internalization ability (FIG. 9).

### Example 6: Invasion test

This experiment was conducted to confirm the inhibitory effect of the B7-H3/TGFβ bispecific molecules on cancer cell invasion.

### 6.1 Preparation of reagents

Culture medium: It was prepared by putting 50 mL of FBS, 5 mL of antibiotic-antimycotic (100 X), 5 mL of non-essential amino acid (NEAA), and 5 mL of sodium pyruvate into 500 mL of RPMI 1640 medium. 1 × PBS: It was prepared by mixing 100 mL of 10 × PBS in 900 mL of tertiary distilled water. 0.2% crystal violet: After putting 10 mL of 1% crystal violet solution to 40 mL of methanol and mixing by inverting, the mixture was stored at room temperature in a light-shielding state.

### 6.2. Transwell insertion and matrigel coating

Forceps were heated with an alcohol lamp and allowed to cool. Transwell was mounted on an SPL 24-well plate. After dispensing 22 μL of matrigel diluted at a ratio of 1:10 with serum free media (SFM) into each insert well (inside the transwell), allowed to be spread evenly on the membrane. Thereafter, the matrigel was dried at room temperature for 1-2 hours to harden.

### 6.3. Seeding and cultivation of RKO, RKO/B7H3 cell lines

After removing the medium of RKO and RKOB7H3 cultured in a 10 cm dish, washing with 8 mL of DPBS, 1 mL of trypsin-EDTA (T/E) solution was put, and left in an incubator at 37 °C for 2-3 minutes to remove the cells. The removed cells were collected in a 15 mL tube using 6 mL of SFM, and then centrifuged at 700 rpm for 3 minutes. After removing the supernatant and dissolving the cell pellet with 3 mL of SFM, the number of cells was counted. SFM was added to make the cell concentration be $5 \times 10^6$ cells/mL.

RKO and RKO/B7H3 were slowly put into an insert well by $1 \times 10^6$ cells/200 μL, respectively, and 600 μL of culture medium supplemented with 10% FBS was added to an outer well.

In order to investigate the antibody treatment effect, RKO/B7H3 ($2 \times 10^5$ cells/200 μL) cell line was mixed with #1 to #9 antibodies at a concentration of 20 μg/mL, respectively, slowly put into the insert well, and 600 μL of culture medium supplemented with 10% FBS was added to the outer well. Thereafter, the cells were cultured for 48 hours in an incubator at 37 °C and 5% $CO_2$.

### 6.4. Crystal violet staining

600 μL of 1 × PBS, 0.2% crystal violet, and tertiary distilled water were dispensed into each well of a 24-well plate.

The cultured cells were taken out and the insert well was turned upside down to remove the medium inside, and then immersed in PBS and washed. Then, the insert well was put into 0.2% crystal violet and stained at room temperature for 30 minutes.

After taking out the insert well and turning it upside down to remove the crystal violet inside, dyeing was stopped by immersing it in tertiary distilled water.

After holding the insert well with forceps and washing it by shaking in the tertiary distilled water contained in a wide bucket, a cotton swab was used to wipe out cells that were not invaded into the inner membrane.

### 6.5. Photography and data analysis

The degree of cell invasion was photographed, and the number of cells invaded was counted using Image J.

## Results

Cancer cell invasion was actively progressed in RKO/B7H3 cell line without any treatment (Non-treat in FIG. 10). On the other hand, when RKO/B7H3 cell line was treated with #1 to #9 bispecific molecules and cultured, invasion was reduced (FIG. 10). If B7-H3 is over-expressed, cancer cell invasion may be actively proceeded but the invasion is inhibited by B7-H3 antibody. In particular, it was confirmed that #1 to #9 bispecific molecules have excellent invasion inhibitory effects.

## Example 7: Migration test

This experiment was conducted to confirm the inhibitory effect of B7-H3 bispecific molecule on cancer cell migration.

## Experimental method

### 7.1. Preparation of reagents

Culture medium: It was prepared by putting 50 mL of FBS, 5 mL of antibiotic-antimycotic (100 X), 5 mL of NEAA, and 5 mL of sodium pyruvate into 500 mL of RPMI 1640 medium. 1 × PBS: It was prepared by mixing 100 mL of 10 × PBS in 900 mL of tertiary distilled water. 0.2% crystal violet: After putting 10 mL of 1% crystal violet solution into 40 mL of methanol and mixing by inverting, the mixture was stored at room temperature in a light-shielding state.

### 7.2. Transwell insertion

Forceps were heated with an alcohol lamp and allowed to cool, and then transwell was mounted on an SPL 24-well plate as much as the amount used.

### 7.3. RKO, RKO/B7H3 seeding and cultivation

After removing the medium of RKO and RKOB7H3 cultured in a 10 cm dish, and washing with 8 mL of DPBS, 1 mL of T/E solution was put, and left in an incubator at 37 °C for 2-3 minutes to remove the cells. The removed cells were collected in a 15 mL tube using 6 mL of SFM, and then centrifuged at 700 rpm for 3 minutes. After removing the supernatant and dissolving the cell pellet with 3 mL of SFM, the number of cells was counted. SFM was added to make the cell concentration be $1 \times 10^6$ cells/mL, and cells were slowly added at a concentration of $2 \times 10^5$ cells/200 $\mu$L to an insert well, then 600 $\mu$L of culture medium supplemented with 10% FBS was added to an outer well. Thereafter, the cells were cultured for 16 hours in an incubator at 37 °C and 5% $CO_2$.

### 7.4. Crystal violet staining

600 $\mu$L of PBS, 0.2% crystal violet, and tertiary distilled water were dispensed into each well of a 24-well plate. The cultured cells were taken out and the insert well was turned upside down to remove the medium inside, and then immersed in PBS and washed. Then, the insert well was put into 0.2% crystal violet and stained at room temperature for 30 minutes. After taking out the insert well and turning it upside down to remove the crystal violet inside, dyeing was stopped by immersing it in tertiary distilled water. After holding the insert well with forceps and washing it by shaking in the tertiary distilled water contained in a wide bucket, a cotton swab was used to wipe out cells that were not migrated into the inner membrane.

### 7.5. Photography

The degree of cancer cell migration was confirmed using a microscope, then the cells were photographed.

### 7.6. Crystal violet extraction

After adding 200 $\mu$L of 100% methanol to each of new 24 wells, the insert well that had been photographed was inserted, and 100 $\mu$L of 100% methanol was added to inside the insert well, then sealed with parafilm and shaken at room temperature for 1 hour to extract the dyed reagent. The insert well was removed, then 200 $\mu$L was scooped out and transferred to a 96-well plate, and the OD values were measured at 590 nm. The degree of migration was compared with the measured OD values.

### 7.7. Data analysis

In the case of analyzing the OD values obtained by crystal violet extraction, the OD values of the experimental group was divided based on the value of non-treated RKO/B7H3 cells, and the degree of migration was converted into a percentage value and compared.

### Results

In RKO/B7H3 cell line without any treatment (Non-treat in FIG. 11), cancer cell migration was actively proceeded. On the other hand, when RKO/B7H3 cell line was treated with #1 to #9 bispecific molecules and cultured, cancer cell migration was reduced (FIG. 11). That is, if B7-H3 is over-expressed, cancer cell migration may be actively proceeded, however, may be inhibited by B7-H3 antibody. In particular, it was confirmed that #1 to #9 bispecific molecules have excellent cancer cell migration inhibitory effects.

### Example 8: Confirmation of TGFβ secretion inhibitory effects

In order to investigate extinction effects TGFβ secreted from RKO/B7H3 cells after treatment of B7-H3/TGFβ bispecific molecules (#1 to #9 bispecific molecules), this experiment was conducted.

### Experimental method

#### 8.1. Preparation of reagents

1 × PBS, washing buffer (1 × PBS-T (0.05% Tween 20)), blocking buffer (1% BSA in 1 × PBS-T (0.05% Tween 20)), antibody dilution buffer, and neutralization buffer were prepared. The antibody dilution buffer used herein was the same as the buffer used as the washing buffer. Further, the neutralization buffer was prepared by adding 25 ml of 1 M HEPES, 12 ml of 5 N NaOH and 13 ml of tertiary distilled water, and mixing the same.

#### 8.2. RKO/B7H3 cell line culture, candidate antibody treatment and supernatant harvesting

After dispensing RKO/B7H3 cells into a 24-well plate by $1 \times 10^5$ cells per well, it was cultured for 24 hours in an incubator at 37 °C and 5% $CO_2$. The medium was removed, SFM was dispensed into each well by 200 μL and removed. Then, SFM was dispensed into each well by 500 μL and cultured for 48 hours in an incubator at 37 °C and 5% $CO_2$. After cell culturing for 48 hours, the cells were treated with B7-H3/TGFβ bispecific molecules (#1 to #9 bispecific molecules) (20 nM), followed by culturing for 24 hours. 24 hours after antibody treatment, the supernatant entered 1.5 ml tube and 300 g of the supernatant in the tube was centrifuged for 3 minutes. Then, 400 μL of the supernatant was gathered in 1.5 ml tube and stored at 80 °C.

#### 8.3. Capture antibody coating (100 μL/well; 2 μg/ml)

Human TGFβ1 capture antibody (stock concentration: 240 μg/mL, -20 °C) was dissolved, then diluted at a ratio of 1:120 using 1 × PBS so as to be a concentration of 2 μg/mL. Thereafter, 0.2 μg/well (100 μL/well) was dispensed into each 96-well plate, and then reacted overnight at room temperature. Then, each well was washed using a washing buffer, and 250 μL/well was dispensed into each well, and then reacted at room temperature for 2 hours.

#### 8.4. ELISA assay

1) Standard preparation: after diluting Human TGFβ1 (stock concentration: 190 ng/ml, -20 °C) at a ratio of 1:95 with an antibody dilution buffer, 2-fold serial dilution was performed from a concentration of 2,000 pg/ml thus to prepare Human TGFβ1 standard.
2) The samples (a treatment group of RKO/B7H3 culture supernatant obtained above with #1 to #9 bispecific molecules, and control (Non-treat)) were each dispensed into a 96-well plate by 100 μL, followed by adding 20 μL of 1 N HCl and then shaking the plate for 10 seconds. Thereafter, a reaction was performed at room temperature for 10 minutes, 1.2 N NaOH/0.5 M HEPES was added by 20 μL to neutralize the product.
3) After dispensing the standard and samples prepared in step 1) into wells by 100 μL per well, it was reacted at room temperature for 2 hours. Thereafter, the product was washed three times using a washing solution.

4) A detection antibody (TGF-beta1 Biotinylated Antibody, R&D Systems, Cat# BAF240), stock concentration: 3 μg/ml, 20 °C) was diluted at a ratio of 1: 60 using an antibody dilution buffer to prepare 50 ng/ml of detection antibody. The diluted detection antibody was dispensed into each well by 100 μL/ml, followed by reacting the same at room temperature for 2 hours. Thereafter, the product was washed three times using a washing solution.

5) 100 μL/ml of streptavidin-HRP diluted at a ratio of 1:40 using the antibody dilution buffer was dispensed into each well, followed by reacting the same at room temperature for 20 minutes in a light-shielding state. Thereafter, the product was washed using a washing solution.

6) 100 μL of TMB was dispensed into each well, followed by reacting the same at room temperature for 20 minutes in a light-shielding state. Thereafter, 50 μL of 1 N HCl as a stop solution was dispensed into each well to terminate a matrix reaction.

7) The optical density (OD) was measured at 450 nm.

## Results

It was confirmed that the immune inhibitory substance, that is, TGFβ1 became completely extinct in the B7-H3/TGFβ bispecific molecules (#1 to #9 bispecific molecules administration group (FIG. 12).

## Example 9: Evaluation of cancer model anticancer efficacy (In vivo efficacy test)

This experiment was conducted to confirm that tumor growth was suppressed in an *in vivo* mouse cancer model when treated with B7-H3/TGFβ bispecific molecules (#1 to #9 bispecific molecules).

### 9.1. Preparation of animal model

Female 7-week-old Balb/c mice (Korea Biolink) were used. Cell line CT26-TN cells prepared by over-expressing B7-H3 in CT26 cells, which are mouse colorectal cancer cell lines, were diluted in DPBS at a concentration of $5 \times 10^6$ cells/mL, and 100 μL ($5 \times 10^5$ cells) per individual were subcutaneously implanted on the right flank. On the 7th day after implanting the cell line, the tumor volume was calculated using the following equation by means of an electronic caliper.

$$\text{Tumor volume (mm}^3) = <\text{Length (mm) x Width (mm)}^2> \text{ x } 0.5$$

### 9.2. Group separation

On the 7th day after implanting the cancer cell line, the implanted right tumor was measured, and when the tumor size of most of the subjects reached about 40-120 mm$^3$, sizes of the implanted tumors on both sides of one subject were measured, and group separation was performed according to the Z array method based on the average value of the tumor sizes.

### 9.3. Antibody administration

Dose concentration: A #5 bispecific molecule administration group - 10 mg/kg of #5 bispecific molecule; and a #5 bispecific molecule and anti-PD-1 antibody combined administration group - 10 mg/kg of #5 bispecific molecule and anti-PD-1 antibody, respectively.

All test substances were administered intravenously (using an insulin syringe) twice a week, for 2 weeks, a total of 4 times, and negative control substances (vehicle (PBS), and IgG) were also administered in the same way.

[TABLE 9]

| Item | Company | Cat No. | Product name |
|------|---------|---------|--------------|
| **Anti-PD-1 antibody** | BioXcell | BE016 | InVivoMab Anti-mouse PD-1 (CD279) |

### 9.4. Measurement of tumor size and weight

After group separation, the sizes of all implanted tumors were measured twice a week for 3 weeks in terms of the tumor volumes. At the same time, the tumor sizes were measured and recorded twice a week after group separation for all animals.

**9.5. Autopsy**

Based on the day of group separation as day 0, tumors were extracted on day 22, photographs were taken for each individual, and tumor weight was measured.

**Results**

The growth of the implanted CT26-TN cell line was rapidly increased in the vehicle (PBS) and IgG administration groups as the negative control, but in the #5 bispecific molecule administration group (G3), it was confirmed that tumor growth was suppressed from the 7th day after regrouping. In addition, it was confirmed that tumor growth was significantly inhibited in the #5 bispecific molecule and anti-PD-1 antibody combined administration group (G4) (BioXcell, Cat# BE016) (FIG. 13).

**Example 10: Quantification of TGF$\beta$ in serum in a mouse cancer model**

This experiment was conducted to confirm TFG$\beta$ changes in serum in an *in vivo* mouse cancer model after H3/TGF$\beta$ bispecific molecules (#1 to #9 bispecific molecules) treatment.

**Experimental method**

**10.1. Material**

Mouse TGF-beta1 DuoSet ELISA kit (Cat#: DY1679)
Serum isolated from mice that had completed the *in vivo* cancer model anticancer test

**10.2. Preparation of antibody**

Mouse TGF$\beta$1 capture antibody was diluted in PBS at a ratio of 1:120, mouse TGF$\beta$1 detection antibody was diluted in PBS at a ratio of 1:60, and streptavidin-HRP was diluted in PBS at a ratio of 1:40.

**10.3. Preparation of serum sample**

10 $\mu$L of 1 N HCl was added to 40 $\mu$L of serum, then shaken at room temperature for 10 seconds and incubated for 10 minutes. Thereafter, 10 $\mu$L of 1.2 N NaOH/0.5 M HEPES was added to stop the reaction, and diluted with PBS at a ratio of 1:2.

**10.4. ELISA assay**

Before one day (before 15 to 18 hours), the diluted capture antibody was dispensed into a 96-well plate by 100 $\mu$L, and cultured at room temperature, followed by washing with 200 $\mu$L of PBS-T (PBS + 0.05% Tween 20).

150 $\mu$L of blocking buffer (PBS+5% Tween 20) was put and cultured for 1 hour at room temperature, followed by washing with 200 $\mu$L of PBS-T. Thereafter, the standard solution provided in the kit and serum sample prepared in advance were dispensed into a 96-well plate by 100 $\mu$L in duplication, and reacted at room temperature for 2 hours, followed by washing three times with 200 $\mu$L of PBS-T. 100 $\mu$L of detection antibody was dispensed into wells, and reacted at room temperature for 2 hours, followed by washing three times with 200 $\mu$L of PBS-T. 100 $\mu$L of streptavidin-HRP was dispensed into wells, and reacted at room temperature for 20 minutes, followed by washing three times with 200 $\mu$L of PBS-T. After dispensing 100 $\mu$L of TMB solution, the color development reaction was performed at room temperature in a light-shielding state. Then, 50 $\mu$L of 1 N HCl was put to stop the color development reaction. Finally, the OD values were measured at 450 nm.

**Results**

It was confirmed that the concentrations of TGF$\beta$ in mouse serum in the #5 bispecific molecule administration group (G3) and the #5 bispecific molecule and anti-PD-1 antibody combined administration group (G4) were decreased significantly (based on the vehicle group, p value < 0.5) compared to the vehicle (PBS) administration group (G1) and IgG administration group (G2) as the negative control (see FIG. 14).

### Example 11: Tumor infiltrating lymphocytes (TIL) Assay

To confirm whether a penetration ability of lymphocytes, as immune cells, into cancer tissues is increased or not when treating *in vivo* mouse cancer models with B7-H3/TGFβ bispecific molecules (#1 to #9 bispecific molecules), this experiment was conducted.

### Experimental method

### 11.1. Tumor cell isolation

10 mL of DPBS was added to tumors extracted from mice that had completed the *in vivo* cancer model anticancer test and washed, then the remaining blood was removed.

6 mL of RPMI-1640 (Hyclone, Cat# CM058-050) medium was put, and cut finely with scissors, then 600 μL of digestion solution (50 mL RPMI-1640 + 100 mg collagenase D (Merck, Cat# 11088858001) + 10 mg DNase I (Sigma-Aldrich, Cat# D4513)) was added, followed by reaction at 37 °C and 120 rpm for 1 hour.

After putting it into a 70 μm cell strainer (SPL, Cat# SPL93070) and filtering large tissues, 1 mL thereof was placed in a 15 mL tube and centrifuged at 15 °C and 2,000 rpm for 10 minutes to remove the supernatant. After washing once with DPBS, 500 μL of 1 × RBC lysis buffer (BioLegend, Cat# 420301) diluted in distilled water was added to release the pellet, and the mixture was reacted at room temperature for 5 minutes.

After washing twice in DPBS by the same method as above, the pellet was well dissolved in FACS buffer (DPBS + 1% FBS + 0.1% sodium azide) to prepare cells.

### 11.2. FACS analysis antibody information

BioLegend products was used as antibodies for FACS analysis, and information thereof is shown in Table 10 below.

[TABLE 10]

| Analysis item | Antibody used | Cat# |
|---|---|---|
| CD4 | APC anti-mouse CD4 | 116014 |
| CD8 | APC/Cyanine7 anti-mouse CD8b.2 | 140422 |
| CD3 | FITC anti-mouse CD3 | 100204 |
| CD45 | PE anti-mouse CD45 | 103106 |

### 11.3. Fluorescence staining

For single cells of the tumor isolated according to the cell separation test method, purified rat anti-mouse CD16/CD32 (Mouse BD Fc Block™, BD biosciences, Cat# 553141) was pretreated for 10 minutes to perform FC blocking, and then the antibodies diluted in FACS buffer (DPBS + 1% FBS + 0.1% sodium azide) at a dilution ratio indicated in the provided data sheet, followed by reaction at 4 °C for 1 hour while blocking light.

The cells after completion of the reaction were washed twice using FACS buffer and then fixed using 2% paraformaldehyde (PFA). The stained cells were measured using a flow cytometer (Attune, Thermo Fisher Scientific) and analyzed using FlowJo™ V10 (Flowjo, LLC).

### Results

As a result of FACS analysis on CD4+ and CD8+ T cells of tumors extracted from mice, it was confirmed that the infiltration abilities of CD8+ TIL immune cells into cancer tissues were increased in the #5 bispecific molecule administration group (G3) and the #5 bispecific molecule and anti-PD-1 antibody combined administration group (G4) compared to the vehicle (PBS) administration group (G1) and IgG administration group (G2). In contrast, it was confirmed that there was no difference in CD4+ T cells between the negative control and the antibody administration groups. From this, it can be seen that cytotoxic lymphocytes (CD8+ T cells) can infiltrate into the cancer tissues to exhibit cytotoxic effects on the cancer cells (see FIG. 15).

**Claims**

1. Abispecific molecule, comprising: a B7-H3 antibody or antigen-binding fragment thereof; and a TGFβ binding portion bound thereto.

2. The bispecific molecule according to claim 1, wherein the B7-H3 antibody or antigen-binding fragment thereof comprises a heavy chain variable region comprising HCDRs below and a light chain variable region comprising LCDRs below:

   (a) HCDRs of SEQ ID NOs: 1, 10 and 19 and LCDRs of SEQ ID NOs: 28, 37 and 45;
   (b) HCDRs of SEQ ID NOs: 2, 11 and 20 and LCDRs of SEQ ID NOs: 29, 38 and 46;
   (c) HCDRs of SEQ ID NOs: 3, 12 and 21 and LCDRs of SEQ ID NOs: 30, 39 and 47;
   (d) HCDRs of SEQ ID NOs: 4, 13 and 22 and LCDRs of SEQ ID NOs: 31, 40 and 48;
   (e) HCDRs of SEQ ID NOs: 5, 14 and 23 and LCDRs of SEQ ID NOs: 32, 41 and 49;
   (f) HCDRs of SEQ ID NOs: 6, 15 and 24 and LCDRs of SEQ ID NOs: 33, 42 and 50;
   (g) HCDRs of SEQ ID NOs: 7, 16 and 25 and LCDRs of SEQ ID NOs: 34, 43 and 51;
   (h) HCDRs of SEQ ID NOs: 8, 17 and 26 and LCDRs of SEQ ID NOs: 35, 44 and 52; or
   (i) HCDRs of SEQ ID NOs: 9, 18 and 27 and LCDRs of SEQ ID NOs: 36, 42 and 53.

3. The bispecific molecule according to claim 1, wherein the TGFβ binding portion is selected from the group consisting of an antibody or antigen-binding fragment thereof, aptamer and TGFβ receptor specifically bound to TGFβ.

4. The bispecific molecule according to claim 1, wherein the TGFβ binding portion is connected by a linker.

5. The bispecific molecule according to claim 1, wherein the TGFβ binding portion comprises an amino acid sequence of SEQ ID NO: 280.

6. The bispecific molecule according to claim 1, wherein the TGFβ binding portion is specifically bound to any one TGFβ selected from the group consisting of TGFβ1, TGFβ2 and TGFβ3.

7. The bispecific molecule according to claim 2, wherein the heavy chain variable region comprises any one framework sequence selected from the group consisting of HFRs below, and the light chain variable region comprises any one framework sequence selected from the group consisting of LFRs below:

   (hf1) HFRs of SEQ ID NOs: 54, 63, 68 and 334;
   (hf2) HFRs of SEQ ID NOs: 55, 63, 69 and 334;
   (hf3) HFRs of SEQ ID NOs: 56, 64, 70 and 334;
   (hf4) HFRs of SEQ ID NOs: 56, 64, 71 and 334;
   (hf5) HFRs of SEQ ID NOs: 57, 64, 70 and 334;
   (hf6) HFRs of SEQ ID NOs: 58, 64, 72 and 334;
   (hf7) HFRs of SEQ ID NOs: 59, 65, 73 and 334;
   (hf8) HFRs of SEQ ID NOs: 60, 65, 73 and 334;
   (hf9) HFRs of SEQ ID NOs: 61, 66, 74 and 334;
   (hf10) HFRs of SEQ ID NOs: 62, 67, 75 and 334;
   (lf1) LFRs of SEQ ID NOs: 76, 82, 86 and 335;
   (lf2) LFRs of SEQ ID NOs: 77, 82, 87 and 335;
   (lf3) LFRs of SEQ ID NOs: 78, 83, 88 and 335;
   (lf4) LFRs of SEQ ID NOs: 79, 84, 89 and 335;
   (lf5) LFRs of SEQ ID NOs: 80, 84, 90 and 335;
   (lf6) LFRs of SEQ ID NOs: 80, 84, 91 and 335;
   (lf7) LFRs of SEQ ID NOs: 81, 85, 92 and 335;
   (lf8) LFRs of SEQ ID NOs: 93, 98, 101 and 336;
   (lf9) LFRs of SEQ ID NOs: 93, 98, 102 and 336;
   (lf10) LFRs of SEQ ID NOs: 93, 98, 103 and 336;
   (lf11) LFRs of SEQ ID NOs: 93, 98, 104 and 336;

(lf12) LFRs of SEQ ID NOs: 94, 98, 105 and 336;
(lf13) LFRs of SEQ ID NOs: 95, 99, 106 and 336;
(lf14) LFRs of SEQ ID NOs: 96, 99, 107 and 336; and
(lf15) LFRs of SEQ ID NOs: 97, 100, 108 and 336.

8. The bispecific molecule according to claim 2, wherein the heavy chain variable region is any one selected from the group consisting of SEQ ID NOs: 127, 128, 129, 130, 131, 132, 135, 142 and 152, and the light chain variable region is any one selected from the group consisting of SEQ ID NOs: 211, 221, 223, 224, 225, 231, 307, 309 and 317.

9. A gene encoding the bispecific molecule according to any one of claims 1 to 8.

10. A cell comprising a vector introduced therein, in which the gene of claim 9 is inserted.

11. A pharmaceutical composition for treating or preventing cancer comprising the bispecific molecule according to any one of claims 1 to 8.

12. The pharmaceutical composition according to claim 11, wherein the cancer is any one selected from the group consisting of lung cancer, breast cancer, ovarian cancer, uterine cancer, cervical cancer, glioma, neuroblastoma, prostate cancer, pancreatic cancer, colorectal cancer, colon cancer, head and neck cancer, leukemia, lymphoma, renal cancer, bladder cancer, gastric cancer, liver cancer, skin cancer, brain tumor, cerebrospinal cancer, adrenal tumor, melanoma, sarcoma, multiple myeloma, pancreatic neuroendocrine neoplasm, peripheral nerve sheath tumor and small cell tumor.

13. The pharmaceutical composition according to claim 11, further comprising an immune checkpoint inhibitor selected from the group consisting of PD-1 inhibitor, PD-L1 inhibitor, CTLA4 inhibitor, LAG3 inhibitor, TIM3 inhibitor and TIGIT inhibitor.

14. The pharmaceutical composition according to claim 1l, further comprising a cellular therapeutic agent selected from the group consisting of CAR-T, TCR-T, cytotoxic T lymphocytes, tumor infiltrating lymphocyte, NK and CAR-NK.

15. The bispecific molecule according to any one of claims 1 to 8, wherein the bispecific molecule is used as a medicine.

[FIG. 1]

[FIG. 2]

[FIG. 3]

**RKO/B7H3 cell line**

[FIG. 4]

**TGF-beta 1**

[FIG. 5]

[FIG. 6]

[FIG. 7]

[FIG. 8]

[FIG. 9]

[FIG. 10]

[FIG. 11]

[FIG. 12]

[FIG. 13]

<Changes in tumor volume (7 mice per group)>

<Tumor extraction photographs (3 mice per group)>

[FIG. 14]

[FIG. 15]

<div style="text-align:center">**INTERNATIONAL SEARCH REPORT**</div>

| International application No. |
|---|
| **PCT/KR2022/012859** |

## A. CLASSIFICATION OF SUBJECT MATTER

**C07K 16/28**(2006.01)i; **C07K 16/22**(2006.01)i; **A61P 35/00**(2006.01)i; **A61K 39/00**(2006.01)i

According to International Patent Classification (IPC) or to both national classification and IPC

## B. FIELDS SEARCHED

Minimum documentation searched (classification system followed by classification symbols)

C07K 16/28(2006.01); A61K 39/00(2006.01); C07K 14/71(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: B7-H3, TGF-beta, 항체(antibody), 이중특이적(bispecific), 암(cancer), 치료 (treatment)

## C. DOCUMENTS CONSIDERED TO BE RELEVANT

| Category* | Citation of document, with indication, where appropriate, of the relevant passages | Relevant to claim No. |
|---|---|---|
| X | WO 2019-241625 A1 (ACCELERON PHARMA INC.) 19 December 2019 (2019-12-19)<br>See abstract; pages 51, 71-72, 77, 82 and 114; and claims 1, 4, 86-87, 89 and 92-96. | 1,3-4,6,9-15 |
| A | | 2,5,7-8 |
| X | WO 2011-109789 A2 (THE JOHNS HOPKINS UNIVERSITY) 09 September 2011 (2011-09-09)<br>See abstract; paragraphs [0108], [0161] and [0198]; and claim 1. | 1,3-4,6,9-15 |
| A | KR 10-2016-0119197 A (MERCK PATENT GMBH) 12 October 2016 (2016-10-12)<br>See entire document. | 1-15 |
| A | HUANG, Cheng et al. Combination therapy with B7H3-redirected bispecific antibody and sorafenib elicits enhanced synergistic antitumor efficacy. Theranostics. 21 August 2020, vol. 10, no. 23, pp. 10498-10512.<br>See entire document. | 1-15 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 December 2022** | **06 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/012859**

### C. DOCUMENTS CONSIDERED TO BE RELEVANT

| Category* | Citation of document, with indication, where appropriate, of the relevant passages | Relevant to claim No. |
|---|---|---|
| A | LIND, Hanne et al. Dual targeting of TGF-β and PD-L1 via a bifunctional anti-PD-L1/TGF-βRII agent: status of preclinical and clinical advances. Journal for ImmunoTherapy of Cancer. 2020, vol. 8, e000433, pp. 1-10.<br>    See entire document. | 1-15 |

Form PCT/ISA/210 (second sheet) (July 2022)

**INTERNATIONAL SEARCH REPORT**

International application No.

**PCT/KR2022/012859**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

| International application No. |
| --- |
| **PCT/KR2022/012859** |

| Patent document cited in search report | | | Publication date (day/month/year) | Patent family member(s) | | | Publication date (day/month/year) |
| --- | --- | --- | --- | --- | --- | --- | --- |
| WO | 2019-241625 | A1 | 19 December 2019 | EP | 3807308 | A1 | 21 April 2021 |
| | | | | JP | 2021-526835 | A | 11 October 2021 |
| | | | | US | 2022-0089683 | A1 | 24 March 2022 |
| WO | 2011-109789 | A2 | 09 September 2011 | EP | 2542590 | A2 | 09 January 2013 |
| | | | | EP | 2542590 | B1 | 03 May 2017 |
| | | | | EP | 2542590 | B2 | 01 April 2020 |
| | | | | EP | 3260470 | A1 | 27 December 2017 |
| | | | | EP | 3798237 | A1 | 31 March 2021 |
| | | | | JP | 2013-521311 | A | 10 June 2013 |
| | | | | JP | 2017-043600 | A | 02 March 2017 |
| | | | | JP | 2018-172439 | A | 08 November 2018 |
| | | | | JP | 2021-143189 | A | 24 September 2021 |
| | | | | JP | 6066732 | B2 | 25 January 2017 |
| | | | | JP | 6378262 | B2 | 22 August 2018 |
| | | | | US | 10442860 | B2 | 15 October 2019 |
| | | | | US | 11274156 | B2 | 15 March 2022 |
| | | | | US | 2013-0039911 | A1 | 14 February 2013 |
| | | | | US | 2015-0183881 | A1 | 02 July 2015 |
| | | | | US | 2016-0340430 | A1 | 24 November 2016 |
| | | | | US | 2017-0158770 | A1 | 08 June 2017 |
| | | | | US | 2020-0115455 | A1 | 16 April 2020 |
| | | | | US | 8993524 | B2 | 31 March 2015 |
| | | | | US | 9441044 | B2 | 13 September 2016 |
| | | | | US | 9850306 | B2 | 26 December 2017 |
| KR | 10-2016-0119197 | A | 12 October 2016 | CN | 106103488 | A | 09 November 2016 |
| | | | | CN | 113549159 | A | 26 October 2021 |
| | | | | EP | 3105246 | A2 | 21 December 2016 |
| | | | | EP | 3105246 | B1 | 31 March 2021 |
| | | | | EP | 3889172 | A1 | 06 October 2021 |
| | | | | JP | 2017-506217 | A | 02 March 2017 |
| | | | | JP | 2020-174674 | A | 29 October 2020 |
| | | | | JP | 6731346 | B2 | 29 July 2020 |
| | | | | KR | 10-2363008 | B1 | 16 February 2022 |
| | | | | US | 2015-0225483 | A1 | 13 August 2015 |
| | | | | US | 2018-0002436 | A1 | 04 January 2018 |
| | | | | US | 2020-0055949 | A1 | 20 February 2020 |
| | | | | US | 9676863 | B2 | 13 June 2017 |
| | | | | WO | 2015-118175 | A2 | 13 August 2015 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220052919